# EUROPEAN PATENT APPLICATION

(11) **EP 2 481 812 A1**
(43) Date of publication of application: **01.08.2012**
(21) Application number: 11152645.5
(22) Date of filing: 31.01.2011
(51) Int. Cl.: C12Q 1/68

(54) **Molecular sexing of avian subjects**

(71) Applicant: Westfälische Wilhelms-Universität Münster, 48149 Münster (DE); Landschaftsverband Westfalen-Lippe, 48147 Muenster (DE)
(72) Inventor: Suh, Alexander, 48163, Muenster (DE); Schmitz, Juergen, 37077, Göttingen (DE); Kriegs, Jan Ole, 48161, Muenster (DE); Brosius, Juergen, 48149, Muenster (DE)
(74) Representative: Schiweck, Weinzierl & Koch

(57) **Abstract**

The present invention provides oligonucleotides including amplification primers and probes as well as pairs of said oligonucleotides that are useful in methods and uses for the determination of the sex of an avian subject. Similarly, these oligonucleotides can be used for the preparation of a kit for the determination of the sex of an avian subject. Also, the present invention provides a method for the identification of a pair of oligonucleotides for sexing of an avian subject and a kit comprising such oligonucleotides. The primer pairs bind to CHD1 or NIPBL on the W and Z chromosomes. The inserts amplified are CR1 or hitchcock-related LTR retroposons.

SEQUENCE LISTING

<110>  Universitaetsklinikum Muenster
<110>  Landschaftsverband Westfalen-Lippe

<120>  Molecular sexing of avian subjects

<130>  UKM13759EP

<160>  16

<170>  PatentIn version 3.5

<210>  1
<211>  21
<212>  DNA
<213>  Artificial

<220>
<223>  Oligonucleotide derived from the avian CHD1 gene

<400>  1
cagcagaaat caatccaaga c                                                21

<210>  2
<211>  23
<212>  DNA
<213>  Artificial

<220>
<223>  Oligonucleotide derived from the avian CHD1 gene

<400>  2
cagcccattt aactgataat ctc                                              23

<210>  3
<211>  22
<212>  DNA
<213>  Artificial

<220>
<223>  Oligonucleotide derived from the avian CHD1 gene

<400>  3
gtcctgattt tctcacagat gg                                               22

<210>  4
<211>  20
<212>  DNA
<213>  Artificial

<220>
<223>  Oligonucleotide derived from the avian CHD1 gene

<400>  4
atgatccagt gcttgtttcc                                                  20

<210>  5
<211>  22
<212>  DNA
<213>  Artificial

<220>
<223>  Oligonucleotide derived from the avian NIPBL gene

<400>  5
ttgtcagagt tgctggagat ac                                                22


<210>  6
<211>  23
<212>  DNA
<213>  Oligonucleotide derived from the avian NIPBL gene

<400>  6
aatttgatgg cacataactg tag                                               23


<210>  7
<211>  25
<212>  DNA
<213>  Artificial

<220>
<223>  5' end of hitchcock-related LTR retroposon consensus sequence


<220>
<221>  misc_feature
<222>  (4)..(4)
<223>  v can be a, g or c

<220>
<221>  misc_feature
<222>  (5)..(5)
<223>  h can be a, c or t

<220>
<221>  misc_feature
<222>  (7)..(7)
<223>  r can be g or a

<220>
<221>  misc_feature
<222>  (9)..(9)
<223>  k can be g or t

<220>
<221>  misc_feature
<222>  (11)..(11)
<223>  y can be t or c

<220>
<221>  misc_feature

<222>  (12)..(12)
<223>  y can be t or c

<220>
<221>  misc_feature
<222>  (13)..(13)
<223>  r can be g or a

<220>
<221>  misc_feature
<222>  (18)..(18)
<223>  r can be g or a

<220>
<221>  misc_feature
<222>  (20)..(20)
<223>  r can be g or a

<220>
<221>  misc_feature
<222>  (22)..(22)
<223>  r can be g or a

<220>
<221>  misc_feature
<222>  (25)..(25)
<223>  r can be g or a

<400>  7
tgtvhtrgkt yyrgctgrgr trgar                                          25


<210>  8
<211>  25
<212>  DNA
<213>  Artificial

<220>
<223>  3' end of hitchcock-related LTR retroposon consensus sequence


<220>
<221>  misc_feature
<222>  (1)..(1)
<223>  y can be t or c

<220>
<221>  misc_feature
<222>  (2)..(2)
<223>  r can be g or a

<220>
<221>  misc_feature
<222>  (4)..(4)
<223>  y can be t or c

<220>
<221>  misc_feature
<222>  (5)..(5)

<223>  k can be g or t

<220>
<221>  misc_feature
<222>  (8)..(8)
<223>  y can be t or c

<220>
<221>  misc_feature
<222>  (14)..(14)
<223>  y can be t or c

<220>
<221>  misc_feature
<222>  (17)..(17)
<223>  m can be a or c

<220>
<221>  misc_feature
<222>  (22)..(22)
<223>  v can be a, g or c

<400>  8
yrcykgcygg ggtyaamcca cvaca                                                25


<210>  9
<211>  51
<212>  DNA
<213>  Artificial

<220>
<223>  3' end of CR1 retroposon consensus sequence


<220>
<221>  misc_feature
<222>  (1)..(1)
<223>  r can be g or a

<220>
<221>  misc_feature
<222>  (9)..(9)
<223>  y can be t or c

<220>
<221>  misc_feature
<222>  (10)..(10)
<223>  n can be a, g, c or t

<220>
<221>  misc_feature
<222>  (16)..(16)
<223>  y can be t or c

<220>
<221>  misc_feature
<222>  (18)..(18)
<223>  y can be t or c

<220>
<221>  misc_feature
<222>  (19)..(19)
<223>  y can be t or c

<220>
<221>  misc_feature
<222>  (20)..(20)
<223>  y can be t or c

<220>
<221>  misc_feature
<222>  (21)..(21)
<223>  n can be a, g, c or t

<220>
<221>  misc_feature
<222>  (22)..(22)
<223>  d can be a, g or t

<220>
<221>  misc_feature
<222>  (23)..(23)
<223>  r can be g or a

<220>
<221>  misc_feature
<222>  (28)..(28)
<223>  y can be t or c

<220>
<221>  misc_feature
<222>  (29)..(29)
<223>  y can be t or c

<220>
<221>  misc_feature
<222>  (37)..(37)
<223>  y can be t or c

<220>
<221>  misc_feature
<222>  (38)..(38)
<223>  y can be t or c

<220>
<221>  misc_feature
<222>  (39)..(39)
<223>  n can be a, g, c or t

<220>
<221>  misc_feature
<222>  (40)..(40)
<223>  n can be a, g, c or t

<220>
<221>  misc_feature
<222>  (41)..(41)

<223>  n can be a, g, c or t

<220>
<221>  misc_feature
<222>  (42)..(42)
<223>  n can be a, g, c or t

<220>
<221>  misc_feature
<222>  (43)..(43)
<223>  n can be a, g, c or t

<220>
<221>  misc_feature
<222>  (49)..(49)
<223>  r can be g or a

<400>  9
rgttggacyn gatgaycyyy ndrggtcyyt tccaacyynn nnnattctrt g                51


<210>  10
<211>  22
<212>  DNA
<213>  Artificial

<220>
<223>  oligonucleotide derived from the avian ATP5A1 gene

<400>  10
aaatggttga gttctcttct gg                                                22


<210>  11
<211>  19
<212>  DNA
<213>  Artificial

<220>
<223>  oligonucleotide derived from the avian ATP5A1 gene

<400>  11
tcaggctcca agttcaagg                                                    19


<210>  12
<211>  21
<212>  DNA
<213>  Artificial

<220>
<223>  oligonucleotide derived from the avian NIPBL gene

<400>  12
gtcagcagtt tgtcagagtt g                                                 21


<210>  13
<211>  21

<212>  DNA
<213>  Artificial

<220>
<223>  oligonucleotide derived from the avian NIPBL gene

<400>  13
cagtcactaa tttgatggca c                                                21

<210>  14
<211>  33
<212>  DNA
<213>  Artificial

<220>
<223>  probe spanning the empty retroposon insertion site in intron 9 of
the CHD1W gene

<220>
<221>  misc_feature
<222>  (14)..(14)
<223>  w can be a or t

<220>
<221>  misc_feature
<222>  (20)..(20)
<223>  r can be g or a

<220>
<221>  misc_feature
<222>  (26)..(26)
<223>  r can be g or a

<220>
<221>  misc_feature
<222>  (29)..(29)
<223>  w can be a or t

<400>  14
gcaatggatt tatwcagttr tgatarttwt taa                                   33

<210>  15
<211>  32
<212>  DNA
<213>  artificial

<220>
<223>  probe spanning the empty retroposon insertion site in intron 16 of
the CHD1W gene

<220>
<221>  misc_feature

<222>  (3)..(3)
<223>  y can be t or c

<220>
<221>  misc_feature
<222>  (11)..(11)
<223>  m can be a or c

<220>
<221>  misc_feature
<222>  (18)..(18)
<223>  y can be t or c

<220>
<221>  misc_feature
<222>  (22)..(22)
<223>  r can be g or a

<220>
<221>  misc_feature
<222>  (26)..(26)
<223>  y can be t or c

<400>  15
agycaagaag mcttgatytt trccayttta tc                                    32


<210>  16
<211>  36
<212>  DNA
<213>  Artificial

<220>
<223>  probe spanning the empty retroposon insertion site in intron 16 of
the NIPBLWgene


<220>
<221>  misc_feature
<222>  (3)..(3)
<223>  y can be t or c

<220>
<221>  misc_feature
<222>  (10)..(10)
<223>  s can be g or c

<400>  16
aayctgatts tattcaaata tcctttaata acttta                                36

## Description

### FIELD OF THE INVENTION

The present invention provides oligonucleotides including amplification primers and probes as well as pairs of said oligonucleotides that are useful in methods and uses for the determination of the sex of an avian subject. Similarly, these oligonucleotides can be used for the preparation of a kit for the determination of the sex of an avian subject. Also, the present invention provides a method for the identification of a pair of oligonucleotides for sexing of an avian subject and a kit comprising such oligonucleotides.

### BACKGROUND OF THE INVENTION

Sex separation is an important aspect for all avian species production, for example, for the broiler and essentially all egg layer and turkey production. For broilers and turkeys, sex separation allows a better suited management and feeding according to the needs of both sexes, which are somewhat different than the unisex rearing as done now in most cases.

However, determination of the sex of avian subjects is also important in studies of ecology, evolutionary biology, breeding and conservation. In avian subjects, the absence of juvenile sexual dimorphism makes it rather difficult and sometimes even impossible to determine an avian subject's sex on the basis of external morphology. Similarly, the same problem is pertinent in fully grown individuals of many avian species where adult sexual dimorphism is absent or at least not very pronounced. It is estimated that adult females appear identical to males in over 50% of the world's bird species. Indeed, adults and particularly offspring of many avian species are monomorphic, making determination of sex difficult. Accordingly, all in all, avian subjects are difficult to sex.

Molecular sexing of avian subjects is therefore an attractive means to determine the sex of avian subjects. Unlike mammals with XY heterogamety, birds possess a sex chromosomal system with ZW heterogamety in females. More specifically, chromosome W is the female-specific sex chromosome in avian species; Z is its homologous chromosome. The biological events that resulted in two different, though homologous sex chromosomes are still not completely understood. The prevalent opinion is that the two sex chromosomes have a common autosomal ancestor, and during evolution, selection and chromosomal aberrations such as inversions prevented effective recombination between some regions of these ancestral chromosomes. Given the ZW heterogamety, chromosome W-specific sequences were identified. In particular, nucleic acid probes that hybridize to the DNA of the female-specific W chromosome have led to molecular solutions for sexing some avian species. However, use of such techniques has proven to be difficult and in many cases, their taxonomic range is limited.

Another method proposed has been to analyse W-specific repeat sequences in order to determine the sex of chick embryos. However, this method required separate sexing and control PCR reactions. Still another method applies PCR single-strand conformation polymorphisms (SSCP) (Cortes et al. (1999), J. Vet. Diagn. Invest 11:297-299).

Still further methods have been proposed in WO 96/39505 which are based on the analysis of DNA sequences (introns and exons) encoding two genes located on the Z and W chromosomes of birds.

WO 20054/016812 proposes to analyze a W chromosome specific transcript as the basis for a sex determination method. Specifically, the W-specific transcript is said to be unique to the W chromosome, thereby allowing the identification of a female avian species.

More recently, polymerase chain reaction (PCR) based approaches that have broader taxonomic utility have been developed. For example, sex identification methods have been based upon examining differences in intron size between the female-specific W chromosome and the Z chromosome, which occurs in both sexes (female, ZW; male, ZZ).

Griffiths et al. (1998), Mol. Evolution 7:1071-1075, employ PCR with a single set of primers in order to amplify homologous sections of the Z and W gametolog, thereby incorporating introns whose lengths usually differ. Accordingly, when examined on a gel, there should be a single *CHD1Z* band in males but females have a second, distinctive *CHD1 W* band. However, for some avian species the method of Griffiths et al. does not allow a clear distinction between male and female sex, since the size difference between the amplification products is too small to allow a clear decision. Moreover, for some avian species (e.g., where only one band is amplified of different size in males and females, or where additional random bands are amplified in males) the method of Griffiths et al. requires a "calibration" (using several conspecific birds of known sex) so that it is not universally applicable.

Fridolfsson and Ellegren (1999, J. Avian Biol. 30:116-121) developed a similar approach and made use of a combination of the analysis of intron size difference between sexes and the chromosome specific *CHD1* gene. Specifically, Fridolfsson and Ellegren developed an allegedly universal method for molecular sexing of non-ratite birds by applying highly conserved primers flanking intron 9 of the *CHD1W* and *CHD1Z* gene, thereby making use of a constant size difference between *CHD1W* and *CHD1Z* introns. Female birds are thus characterized by displaying one small *(CHD1W*) or two fragments *(CHD1W* and *CHD1Z),* while males only show one large fragment *CHD1Z).* With one particular pair of primers, Fridolfsson and Ellegren were able to sex 47 out of 50 bird species from 11 orders throughout the avian phylogeny. Yet, in order to sex the failing species, an alternative pair of primers (only suitable for sexing these few species) had to be applied. Accordingly, though Fridolfsson and Ellegren aimed at the provision of a universal method for molecular sexing of birds by the use of so-called universal primers, their method did not prove to be universally applicable.

### SUMMARY OF THE INVENTION

Having regard to the state of the art, there still exists a need for means and methods that overcome the problems in the prior art in the determination of the sex of an avian subject. These means and methods should be applicable for a simple, universally applicable determination of the sex of an avian subject belonging to the neognaths (99% of all bird species). Accordingly, the technical problem of the present invention is to comply with the need set out in the prior art.

The present invention addresses this need and thus provides as a solution to the technical problem embodiments pertaining to means and methods for determining the sex of an avian subject.

In their investigations of the evolution of gametologs (paralogs on opposite sex chromosomes) from the perspective of retroposon insertions and random insertions/deletions for the reconstruction of gametologous gene trees, the present inventors found three loci that can be utilized as universal, easy-to-use and independent tools for molecular sexing of virtually 95% to 99% of all extant birds. More specifically, in order to examine avian sex chromosome evolution from an independent and novel perspective, the present inventors analysed presence/absence patterns of retroposon insertions (mobile elements that propagate and integrate randomly in genomes via RNA intermediates) to reconstruct gene trees of gametologous genes.

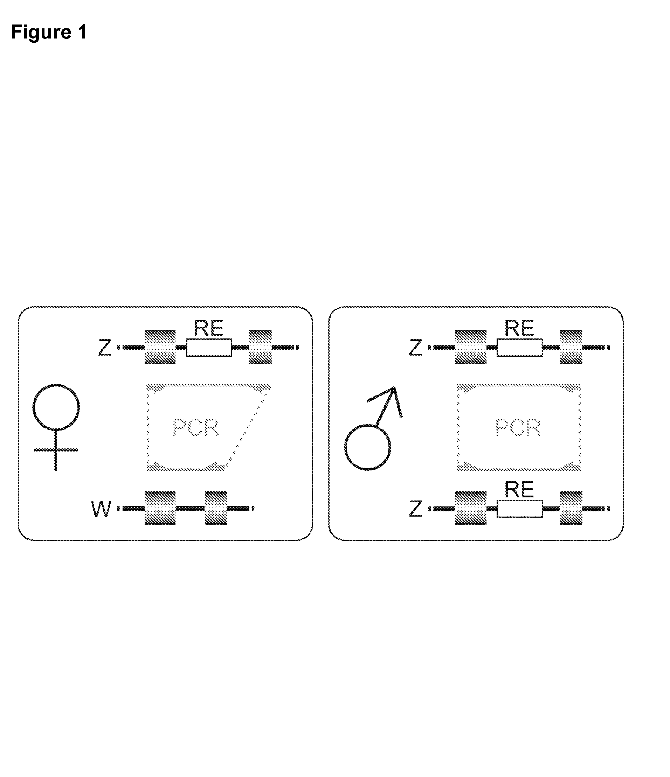

To find gametologous retroposon insertion loci, the chicken (*Gallus gallus*) Z and W chromosomes and the zebra finch (*Taeniopygia guttata*) Z chromosome (assemblies galGal3 and taeGut1 in Genome Browser, http://genome.ucsc.edu/cgi-bin/hgBlat), later also all available gametolog sequences in GenBank (http://www.ncbi.nlm.nih.gov/Genbank/) were scanned for retroposed elements using RepeatMasker (http://www.repeatmasker.org/cgi-bin/WEBRepeatMasker ) and CENSOR (http://www.girinst.org/censor/index.php). For introns (smaller than 1000 bp) present on both sex chromosomes, the chicken Z, chicken W, zebra finch Z and other available homologous sequences were aligned using MAFFT (E-INS-I, version 6, http://mafft.cbrc.jp/alignment/server/) and inspected by eye. The remaining cases feasible for PCR were then used for exonic primer design to amplify intronic sequences that contain a retroposon insertion in the Z gametolog which is, however, absent in the corresponding and thus smaller intron of the W gametolog (Figure 1).

To this end, the present inventors revealed that in introns 9 and 16 of the CHD1 gene (encoding chromo-helicase-DNA-binding protein 1) a retroposon insertion is present in the Z gametolog, but absent in the W gametolog. Similarly, it was found that in intron 16 of the *NIPBL* gene (encoding a homolog of the *Drosophila* Nipped-B protein) another retroposon insertion is solely present in the Z gametolog.

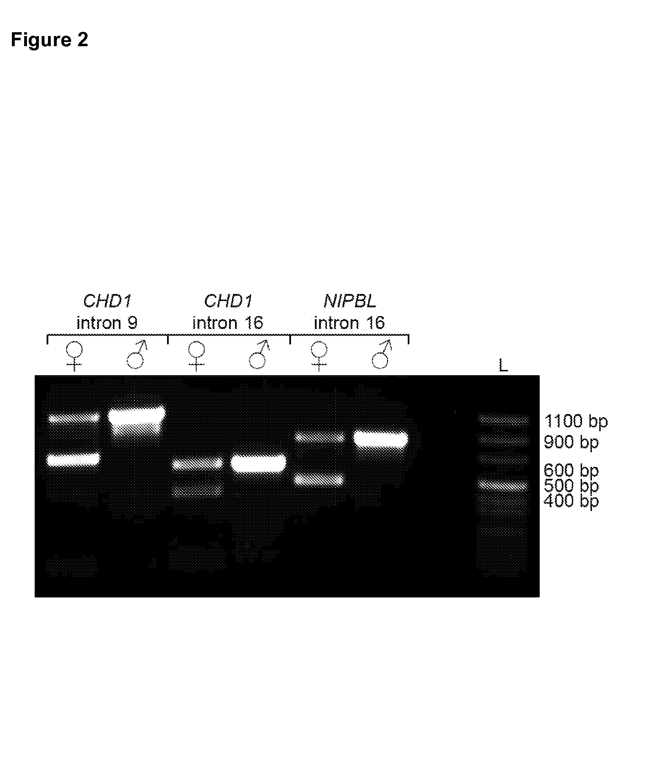

In fact, for example, PCR amplifications of the *CHD1* intron 16 in neognaths and the *NIPBL* intron 16 in neoavians yielded two distinct amplicons in females and only the respective larger amplicon in males. Consequently, retroposon insertions in one of the two gametologs are ideal tools for molecular sexing. Due to the distinct size differences in Z and W amplicons (for example, 200 bp in *CHD1* intron 16, 400 bp in *NIPBL* intron 16 and 500 bp in *CHD1* intron 9; see Figure 2), retroposon sexing markers are not prone to misinterpretation compared to previous markers based on short random indels (e.g., 50 bp in the marker reported by Griffiths et al. 1998, cited above).

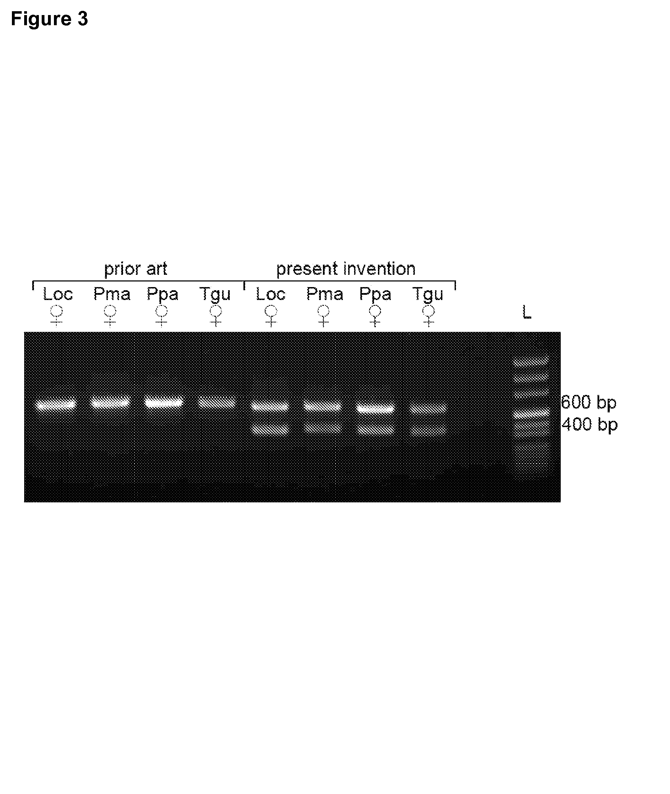

All the more, by the application of the means and methods of the present invention, the inventors could even sex those bird species which failed to be sexed by the means and methods applied by Fridolfsson and Ellegren (1999), cited above (see Figure 3).

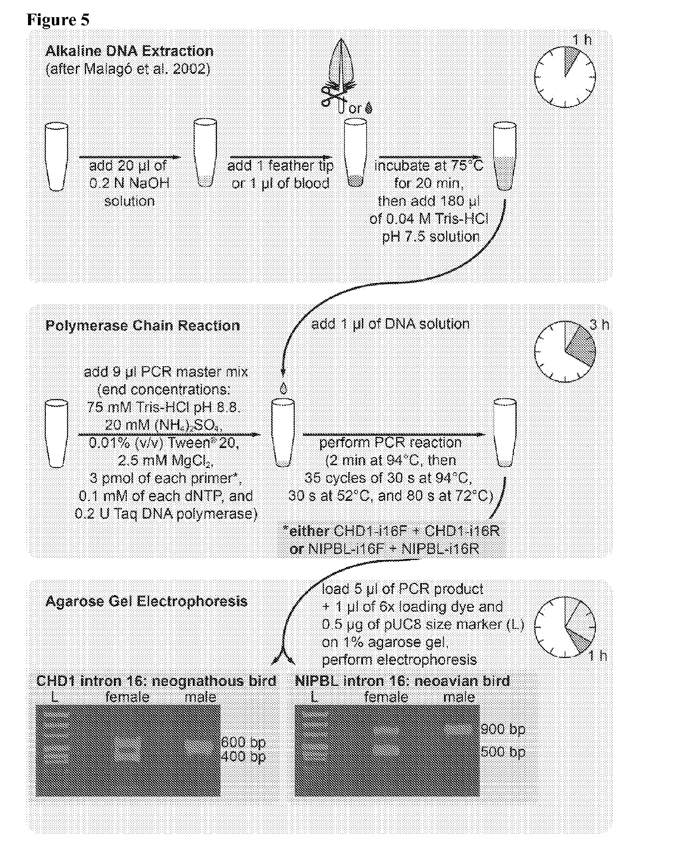

Thus, these three loci *(CHD1* with introns 9 and 11, *NIPBL* with intron 16) will enable the convenient determination of the molecular gender in virtually 95%-99% of all birds using three universal and independent tests (see Figure 5 for a standard operation procedure).

In sum, the present inventors found three loci which, in each case, allow reliable molecular sexing because of a constant and distinct difference in intron size between the female-specific W chromosome and the Z chromosome. Accordingly, the present invention provides ideal tools for molecular sexing of avian subjects.

Aspects of the present invention are
(1) A pair of oligonucleotides selected from the group consisting of:
   (a) a first oligonucleotide comprising at least 10 consecutive nucleotides of the sequence shown in SEQ ID No.1 and a second oligonucleotide comprising at least 10 consecutive nucleotides of the sequence shown in SEQ ID No.2;
   (b) a first oligonucleotide comprising at least 10 consecutive nucleotides of the sequence shown in SEQ ID No.3 and a second oligonucleotide comprising at least 10 consecutive nucleotides of the sequence shown in SEQ ID No.4;
   (c) a first oligonucleotide comprising at least 10 consecutive nucleotides of the sequence shown in SEQ ID No.5 and a second oligonucleotide comprising at least 10 consecutive nucleotides of the sequence shown in SEQ ID No.6; and
   (d) a first oligonucleotide comprising at least 10 consecutive nucleotides of the sequence shown in SEQ ID No.12 and a second oligonucleotide comprising at least 10 consecutive nucleotides of the sequence shown in SEQ ID No.13.
   wherein said pair of oligonucleotides is capable of amplifying a Z- and W-gametologous sequence from one or more species selected from the group consisting of *Chloebia gouldiae, Locustella ochotensis, Parus major, Parus palustris,* and *Taeniopygia guttata.*
(2) The pair of oligonucleotides of item (1), wherein each of said oligonucleotides has a length of not more than 40 nucleotides.
(3) The pair of oligonucleotides of any one of the preceding items, wherein
   (a) said first oligonucleotide as referred to in (a) comprises the nucleotide sequence shown in SEQ ID No.1 and said second oligonucleotide as referred to in (a) comprises the nucleotide sequence shown in SEQ ID No.2;
   (b) said first oligonucleotide as referred to in (b) comprises the nucleotide sequence shown in SEQ ID No.3 and said second oligonucleotide as referred to in (b) comprises the nucleotide sequence shown in SEQ ID No.4;
   (c) said first oligonucleotide as referred to in (c) comprises the nucleotide sequence shown in SEQ ID No.5 and said second oligonucleotide as referred to in (c) comprises the nucleotide sequence shown in SEQ ID No.6;
   (d) said first oligonucleotide as referred to in (d) comprises the nucleotide sequence shown in SEQ ID No.12 and said second oligonucleotide as referred to in (d) comprises the nucleotide sequence shown in SEQ ID No.13
(4) The pair of oligonucleotides of any one of the preceding items, wherein said Z-gametologous sequence amplified by the pair of oligonucleotides
   - of (a) comprises at least 10 consecutive nucleotides of the sequence shown in SEQ ID No.7 or 8;
   - of (b), (c) or (d) comprises at least 10 consecutive nucleotides of the sequence shown in SEQ ID No.9.
(5) The pair of oligonucleotides of any one of the preceding items, wherein said oligonucleotides are further capable of amplifying a Z- and W-gametologous sequence from one or more avian species belonging to the group consisting of the following avian orders: Anseriformes, Apodiformes, Caprimulgiformes, Charadriiformes, Ciconiiformes, Coliiformes, Columbiformes, Coraciiformes, Cuculiformes, Falconiformes, Galbuliformes, Galliformes, Gaviiformes, Gruiformes, Opisthocomiformes, Passeriformes, Pelecaniformes, Piciformes, Phoenicopteriformes, Podicipediformes, Procellariiformes, Psittaciformes, Pterocliformes, Sphenisciformes, Strigiformes, Trogoniformes (*sensu* del Hoyo et al. (Handbook of Birds of the World, Volume 1: Ostrich to Ducks, J. del Hoyo, A. Elliott, and J. Sargatal, Eds., 1992, Lynx Edicions, Barcelona, Spain).
(6) The pair of oligonucleotides of any one of the preceding items, wherein each of said oligonucleotides is labeled with a detectable label.
(7) The pair of oligonucleotides of item (6), wherein said detectable label is selected from the group consisting of luminescent markers, fluorescent markers, radioactive markers and enzyme markers.
(8) A method for the determination of the sex of an avian subject, comprising
   (a) contacting a nucleotide sequence comprised in a sample from said subject with a pair of oligonucleotides referred to in any one of the preceding items;
   (b) amplifying said nucleotide sequence;
   (c) determining the sex of said avian subject by the presence of amplification products of the same size or amplification products of two different sizes,
   wherein amplification products of the same size are indicative of a male sex and amplification products of two different sizes are indicative of a female sex.
(9) The method of item (8), wherein said nucleic acids are amplified by an amplification technique selected from the group consisting of PCR, nested PCR, multiplex PCR, ligase chain reaction (LCR) , transcription-based amplification systems (TAS), self-sustained sequence replication (SSR), nucleic acid sequence-based amplification (NASBA), strand displacement amplification (SDA) or branched DNA (bDNA).
(10) The method of claim (8) or (9), wherein in the amplification step a probe specific for one or more of the sequences shown in SEQ ID Nos.7-9 is present.
(11) The method of any one of items (8-10), wherein said sample preferably is a feather tip or feather pulp.
(12) Use of a pair of oligonucleotides referred to in any one of the preceding items for the determination of the sex of an avian subject.
(13) Use of a pair of oligonucleotides referred to in any one of the preceding items for the preparation of a kit for the determination of the sex of an avian subject.
(14) A method for the identification of a pair of oligonucleotides for sexing of an avian subject, comprising the following steps:
   (a) identifying Z-gametologous sequences which comprise CR1 retroposon and/or *hitchcock*-related LTR retroposon sequences;
   (b) aligning said Z-gametologous sequences identified in (a) with corresponding W-gametologous sequences;
   (c) determining whether said aligned W-gametologous sequences lack the CR1 and/or *hitchcock*-related LTR retroposon sequences; and
   (d) providing a pair of oligonucleotides, wherein each oligonucleotide is substantially complementary to a region that is 5' and 3', respectively, of said aligned sequences and which is capable of simultaneously amplifying both a W-gametologous sequence and a Z-gametologous sequence, the latter comprising CR1 retroposon and/or hitchcock-related LTR retroposon sequence.
(15) The method of item (14), further comprising step (e) determining whether said pair of oligonucleotides is capable of amplifying a W-gametologous sequence from one or more species selected from the group consisting of *Chloebia gouldiae, Locustella ochotensis*, *Parus major, Parus palustris* and *Taeniopygia guttata.*
(16) Pair of oligonucleotides for sexing of an avian subject obtainable by the method of item (14) or (15).
(17) A method for the identification of a probe for sexing of an avian subject, comprising:
   (a) identifying Z-gametologous sequences which comprise CR1 retroposon and/or *hitchcock*-related LTR retroposon sequences;
   (b) aligning said Z-gametologous sequences identified in (a) with corresponding W-gametologous sequences;
   (c) determining whether said aligned W-gametologous sequences lack the CR1 and/or *hitchcock*-related LTR retroposon sequences; and
   (d) providing a probe, wherein said probe is substantially complementary to a nucleotide sequence that spans the nucleotide sequence in the W gametolog that lacks the retroposon insertion in comparison to the Z gametolog.
(18) A probe obtainable by the method of item (17).
(19) A kit for the determination of the sex of an avian subject comprising at least the pair of oligonucleotides of any one of items (1)-(7) or (16) and/or a probe of item (18).
(20) The kit of item (19) further comprising an oligonucleotide probe which is capable of hybridizing to a CR1 retroposon and/or hitchcock-related LTR retroposon sequence.
(21) The kit of item (19) or (20), wherein said oligonucleotide probe has a length of 10-50 nucleotides.
(22) The kit of any one of items (19)-(21) further comprising means for performing an amplification technique.

It must be noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

All publications and patents cited in this disclosure are incorporated by reference in their entirety. To the extent the material incorporated by reference contradicts or is inconsistent with this specification, the specification will supersede any such material.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or sometimes when used herein with the term "having".

When used herein "consisting of" excludes any element, step, or ingredient not specified in the claim element. When used herein, "consisting essentially of" does not exclude materials or steps that do not materially affect the basic and novel characteristics of the claim.
In each instance herein any of the terms "comprising", "consisting essentially of" and "consisting of" may be replaced with either of the other two terms.

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or" as used herein.

As described herein, "preferred embodiment" means "preferred embodiment of the present invention". Likewise, as described herein, "various embodiments" and "another embodiment" means "various embodiments of the present invention" and "another embodiment of the present invention", respectively.

Several documents are cited throughout the text of this specification. Each of the documents cited herein (including all patents, patent applications, scientific publications, manufacturer's specifications, instructions, etc.), whether supra or infra, are hereby incorporated by reference in their entirety. Nothing herein is to be construed as an admission that the invention is not entitled to antedate such disclosure by virtue of prior invention.

### SUMMARY OF THE INVENTION

As mentioned herein above, the present inventors found that introns 9 and 16 of the *CHD1* gene and intron 16 of the *NIPBL* gene are particularly useful for the determination of the sex of an avian subject, since these introns contain retroposed elements in the Z chromosome introns, while these retroposed elements are lacking in the corresponding introns of the W chromosome.

Given the above, in a first aspect the present invention provides a pair of oligonucleotides selected from the group consisting of:
(a) a first oligonucleotide comprising at least 10 consecutive nucleotides of the sequence shown in SEQ ID No.1 and a second oligonucleotide comprising at least 10 consecutive nucleotides of the sequence shown in SEQ ID No.2;
(b) a first oligonucleotide comprising at least 10 consecutive nucleotides of the sequence shown in SEQ ID No.3 and a second oligonucleotide comprising at least 10 consecutive nucleotides of the sequence shown in SEQ ID No.4;
(c) a first oligonucleotide comprising at least 10 consecutive nucleotides of the sequence shown in SEQ ID No.5 and a second oligonucleotide comprising at least 10 consecutive nucleotides of the sequence shown in SEQ ID No.6; and
(d) a first oligonucleotide comprising at least 10 consecutive nucleotides of the sequence shown in SEQ ID No.12 and a second oligonucleotide comprising at least 10 consecutive nucleotides of the sequence shown in SEQ ID No.13.
wherein said pair of oligonucleotides is capable of amplifying a Z- and W-gametologous sequence from one or more species selected from the group consisting of *Chloebia gouldiae, Locustella ochotensis, Parus major, Parus palustris* and *Taeniopygia guttata.*

A Z-gametologous sequence is a sequence from the Z gametolog (or paralog on the Z chromosome) of an avian subject. Similarly, a W-gametologous sequence is a sequence from the W gametolog (or paralog on the W chromosome) of an avian subject.

In addition to the pair of oligonucleotides, the present invention also provides for oligonucleotides that comprise, consist essentially of, or consist of at least 10 consecutive nucleotides of the sequences set forth in SEQ ID Nos: 1-6 or the complement thereof.

The nucleotide sequence shown in SEQ ID No.1 (5'-CAGCAGAAAT CAATCCAAGA C-3') is an oligonucleotide that can preferably be used as forward primer for the amplification of a nucleotide sequence of the *CHD1* gene, in particular for the amplification of a part of exon 9 and intron 9 (including a retroposed element as described herein).

The nucleotide sequence shown in SEQ ID No.2 (5'-CAGCCCATTT AACTGATAAT CTC-3') is an oligonucleotide that can preferably be used as reverse primer for the amplification of a nucleotide sequence of the *CHD1* gene, in particular for the amplification of a part of exon 10 and intron 9 (including a retroposed element as described herein).

The nucleotide sequence shown in SEQ ID No.3 (5'-GTCCTGATTTTCTCACAGATGG-3') is an oligonucleotide that can preferably be used as forward primer for the amplification of a nucleotide sequence of the *CHD1* gene, in particular for the amplification of a part of exon 16 and intron 16 (including a retroposed element as described herein).

The nucleotide sequence shown in SEQ ID No.4 (5'-ATGATCCAGTGCTTGTTTCC-3') is an oligonucleotide that can preferably be used as reverse primer for the amplification of a nucleotide sequence of the *CHD1* gene, in particular for the amplification of a part of exon 17 and intron 16 (including a retroposed element as described herein).

The nucleotide sequence shown in SEQ ID No.5 (5'-TTGTCAGAGTTGCTGGAGATAC-3') is an oligonucleotide that can preferably be used as forward primer for the amplification of a nucleotide sequence of the *NIPBL* gene, in particular for the amplification of a part of exon 16 and intron 16 (including a retroposed element as described herein).

The nucleotide sequence shown in SEQ ID No.6 (5'-AATTTGATGGCACATAACTGTAG-3') is an oligonucleotide that can preferably be used as reverse primer for the amplification of a nucleotide sequence of the *NIPBL* gene, in particular for the amplification of a part of exon 17 and intron 16 (including a retroposed element as described herein).

The nucleotide sequence shown in SEQ ID No.12 (5'-GTCAGCAGTTTGTCAGAGTTG-3') is an oligonucleotide that can preferably be used as forward primer for the amplification of a nucleotide sequence of the *NIPBL* gene, in particular for the amplification of a part of exon 16 and intron 16 (including a retroposed element as described herein).
The nucleotide sequence shown in SEQ ID No.13 (5'-CAGTCACTAATTTGATGGCAC-3') is an oligonucleotide that can preferably be used as reverse primer for the amplification of a nucleotide sequence of the *NIPBL* gene, in particular for the amplification of a part of exon 17 and intron 16 (including a retroposed element as described herein).

Of note, the nucleotide sequence of the aforementioned forward primers is depicted in 5'→3' direction and is thus, so to say, a copy of the plus (+) strand of a double stranded nucleic acid molecule. Accordingly, it is also complementary to the minus (-) or lower strand of a double stranded nucleic acid molecule.
Likewise, the nucleotide sequence of the aforementioned reverse primers is depicted in 5'→3' direction and is the reverse complement of the plus (+) strand of a double stranded nucleic acid molecule. Accordingly, it is also complementary to the plus (+) or upper strand of a double stranded nucleic acid molecule.

Oligonucleotides or pairs of said oligonucleotides disclosed herein can preferably be applied in a method or use for the determination of the sex of an avian subject, preferably in a sample from said avian subject.

In various preferred embodiment said oligonucleotides, in particular oligonucleotide primers, comprise, consist essentially of, or consist of at least 10, 11, 12, 13, 14, 15, 16, 17, 18, 19, 20, 21, 22, 23, 24, 25, 26, 27, 28, 29, 30, 35, 40, 45 or 50 consecutive nucleotides of the sequences set forth in SEQ ID Nos: 1-6.

In some preferred embodiments, an oligonucleotide, in particular an oligonucleotide primer, of the present invention has a length of not more than 30, 35 or 40 nucleotides.

In some other preferred embodiments, an oligonucleotide, in particular an oligonucleotide probe, of the present invention has a length of at least 10 nucleotides or is no longer than 100 nucleotides. For example, a probe has a length of 10-100 nucleotides, preferably a length of 12-100 nucleotides such as 12, 13, 14, 15, 16, 17, 18, 19 or 20 nucleotides, more preferably a length of 15-100 nucleotides, even more preferably a length of 20-100 nucleotides such as 20, 25, 30, 35, 40, 45, 50, 55, 60, 65, 70, 75, 80, 85, 90, 95, or 100 nucleotides.

In a particular preferred embodiment, said oligonucleotides, in particular oligonucleotide primers, comprise, consist essentially of, or consist of the sequence shown in any one of SEQ ID Nos.1-6.

Preferably, oligonucleotides of the present invention are in the form of (a) pair(s) of oligonucleotides (first and second oligonucleotide). Sometimes herein the term "pair of oligonucleotides" is interchangeably used with the term "set of oligonucleotides. For example, a set of oligonucleotides will comprise of at least two primers, one 'upstream' and one 'downstream' primer which together define the amplicon (or amplification product, i.e., the sequence that will be amplified using said primers). These pairs (or sets) of oligonucleotides are preferably applied in the methods, assays or kits of the present invention.

Particularly preferred, a pair of oligonucleotides is applied for the amplification of a nucleotide sequence of a nucleic acid. Said nucleic acid is preferably from an avian subject.

In some preferred embodiments, a first oligonucleotide comprising at least 10 consecutive nucleotides of the sequence shown in SEQ ID No.1 and a second oligonucleotide comprising at least 10 consecutive nucleotides of the sequence shown in SEQ ID No.2 is a preferred pair of oligonucleotides of the present invention.

In some other preferred embodiments, a first oligonucleotide comprising at least 10 consecutive nucleotides of the sequence shown in SEQ ID No.3 and a second oligonucleotide comprising at least 10 consecutive nucleotides of the sequence shown in SEQ ID No.4 is a preferred pair of oligonucleotides of the present invention.

In yet some other preferred embodiments, a first oligonucleotide comprising at least 10 consecutive nucleotides of the sequence shown in SEQ ID No.5 and a second oligonucleotide comprising at least 10 consecutive nucleotides of the sequence shown in SEQ ID No.6 is a preferred pair of oligonucleotides of the present invention.

In some alternatively preferred embodiments, a first oligonucleotide comprising at least 10 consecutive nucleotides of the sequence shown in SEQ ID No.12 and a second oligonucleotide comprising at least 10 consecutive nucleotides of the sequence shown in SEQ ID No.13 is a preferred pair of oligonucleotides of the present invention.

In some preferred embodiments, a pair of oligonucleotides comprising a first oligonucleotide comprising at least 10 consecutive nucleotides of the sequence shown in SEQ ID No.1 and a second oligonucleotide comprising at least 10 consecutive nucleotides of the sequence shown in SEQ ID No.2 and a pair of oligonucleotides comprising a first oligonucleotide comprising at least 10 consecutive nucleotides of the sequence shown in SEQ ID No.3 and a second oligonucleotide comprising at least 10 consecutive nucleotides of the sequence shown in SEQ ID No.4 is applied in the methods, assays or kits of the present invention.

In some other preferred embodiments, a pair of oligonucleotides comprising a first oligonucleotide comprising at least 10 consecutive nucleotides of the sequence shown in SEQ ID No.1 and a second oligonucleotide comprising at least 10 consecutive nucleotides of the sequence shown in SEQ ID No.2 and a pair of oligonucleotides comprising a first oligonucleotide comprising at least 10 consecutive nucleotides of the sequence shown in SEQ ID No.5 and a second oligonucleotide comprising at least 10 consecutive nucleotides of the sequence shown in SEQ ID No.6 is applied in the methods, assay or kits of the present invention.

In alternative, but also preferred embodiments, a pair of oligonucleotides comprising a first oligonucleotide comprising at least 10 consecutive nucleotides of the sequence shown in SEQ ID No.1 and a second oligonucleotide comprising at least 10 consecutive nucleotides of the sequence shown in SEQ ID No.2 and a pair of oligonucleotides comprising a first oligonucleotide comprising at least 10 consecutive nucleotides of the sequence shown in SEQ ID No.12 and a second oligonucleotide comprising at least 10 consecutive nucleotides of the sequence shown in SEQ ID No.13 is applied in the methods, assay or kits of the present invention.

In yet some other preferred embodiments, a pair of oligonucleotides comprising a first oligonucleotide comprising at least 10 consecutive nucleotides of the sequence shown in SEQ ID No.3 and a second oligonucleotide comprising at least 10 consecutive nucleotides of the sequence shown in SEQ ID No.4 and a pair of oligonucleotides comprising a first oligonucleotide comprising at least 10 consecutive nucleotides of the sequence shown in SEQ ID No.5 and a second oligonucleotide comprising at least 10 consecutive nucleotides of the sequence shown in SEQ ID No.6 is applied in the methods, assays or kits of the present invention.

In alternatively preferred embodiments, a pair of oligonucleotides comprising a first oligonucleotide comprising at least 10 consecutive nucleotides of the sequence shown in SEQ ID No.3 and a second oligonucleotide comprising at least 10 consecutive nucleotides of the sequence shown in SEQ ID No.4 and a pair of oligonucleotides comprising a first oligonucleotide comprising at least 10 consecutive nucleotides of the sequence shown in SEQ ID No.12 and a second oligonucleotide comprising at least 10 consecutive nucleotides of the sequence shown in SEQ ID No.13 is applied in the methods, assays or kits of the present invention.

In some preferred embodiments, a pair of oligonucleotides comprising a first oligonucleotide comprising at least 10 consecutive nucleotides of the sequence shown in SEQ ID No.1 and a second oligonucleotide comprising at least 10 consecutive nucleotides of the sequence shown in SEQ ID No.2 and a pair of oligonucleotides comprising a first oligonucleotide comprising at least 10 consecutive nucleotides of the sequence shown in SEQ ID No.3 and a second oligonucleotide comprising at least 10 consecutive nucleotides of the sequence shown in SEQ ID No.4 and a pair of oligonucleotides comprising a first oligonucleotide comprising at least 10 consecutive nucleotides of the sequence shown in SEQ ID No.5 and a second oligonucleotide comprising at least 10 consecutive nucleotides of the sequence shown in SEQ ID No.6 is applied in the methods, assays or kits of the present invention.

In alternatively preferred embodiments, a pair of oligonucleotides comprising a first oligonucleotide comprising at least 10 consecutive nucleotides of the sequence shown in SEQ ID No.1 and a second oligonucleotide comprising at least 10 consecutive nucleotides of the sequence shown in SEQ ID No.2 and a pair of oligonucleotides comprising a first oligonucleotide comprising at least 10 consecutive nucleotides of the sequence shown in SEQ ID No.3 and a second oligonucleotide comprising at least 10 consecutive nucleotides of the sequence shown in SEQ ID No.4 and a pair of oligonucleotides comprising a first oligonucleotide comprising at least 10 consecutive nucleotides of the sequence shown in SEQ ID No.12 and a second oligonucleotide comprising at least 10 consecutive nucleotides of the sequence shown in SEQ ID No.13 is applied in the methods, assays or kits of the present invention.

Strikingly, the present inventors have found means and methods that are ideal tools for the determination of the sex of an avian subject. In fact, as opposed to the state of the art, the means and methods of the present invention allow the determination of the sex of those avian species that could not be sexed by prior art methods (see Figure 3). Thus, in addition to the species of the orders that could be sexed by prior art methods, the present invention provides means and methods to also sex avian species that could not be sexed by prior art methods (Fridolfsson and Ellegren, cited above).

To this end, the oligonucleotides and pairs of oligonucleotides of the present invention are preferably capable of amplifying Z- and W-gametologous sequences from one or more avian species belonging to the group consisting of the following avian orders *sensu* del Hoyo et al. (cited above): Anseriformes, Apodiformes, Caprimulgiformes, Charadriiformes, Ciconiiformes, Coliiformes, Columbiformes, Coraciiformes, Cuculiformes, Falconiformes, Galbuliformes, Galliformes, Gaviiformes, Gruiformes, Opisthocomiformes, Passeriformes, Pelecaniformes, Piciformes, Phoenicopteriformes, Podicipediformes, Procellariiformes, Psittaciformes, Pterocliformes, Sphenisciformes, Strigiformes, Trogoniformes.

However, all the more, the oligonucleotides and pairs of oligonucleotides of the present invention are additionally capable of amplifying Z- and W-gametologous sequences from one or more species selected from the group consisting of *Chloebia gouldiae, Locustella ochotensis, Parus major, Parus palustris* and *Taeniopygia guttata,* thereby allowing sexing of one or more of these species.

Moreover, the present invention overcomes a further drawback of the prior art. Namely, prior art methods suffer from the disadvantage in that they require, so to say, every time a new calibration for some avian species that are intended to be sexed (Griffiths et al., cited above). This is so because prior art methods (i) produce amplification products of gametologous sequences that differ in number and size from species to species, making a universal application not feasible or (ii) produce Z and W amplicons that do not differ much in size, thereby making a distinction difficult.

However, the Z- and W-gametologous sequences amplified by the oligonucleotides of the present invention are conveniently distinguishable by their size, thereby allowing an unmistakable sexing of avian subjects (see Figures 2, 3 and 7).

In fact, as opposed to the prior art the oligonucleotides of the present invention are capable of amplifying not only Z-gametologous sequences of one or more of the above species, but also W-gametologous sequences. Hence, while the prior art identifies female specimen as male specimen, the present invention correctly identifies the female sex (see Figure 3). Therefore, the present invention provides more reliable means and methods for the determination of the sex of an avian subject.

Accordingly, the present invention thus contemplates to provide improved means and methods for the determination of the sex of an avian subject. Indeed, in further contrast to the prior art, the present invention provides not less than three loci that can be used for sexing an avian subject. Hence, the present invention provides three independent loci that can be used to reliably sex an avian subject, for example, by performing three independent assays in accordance with the teaching of the present invention, either as single assays or multiplex assay. In addition, the knowledge that retroposons, so to say, make the difference between Z- and W-gametologous sequences, allows the generation of specific probes that preferably detect the female-specific "empty" retroposon insertion site in the W-gametologous sequence and not the Z gametolog, where the probe's binding site is interrupted due to the retroposon insertion. These probes can be used alternatively or in addition to the amplification primers of the present invention, thereby making the determination of the sex of an avian subject even more convenient or even more specific/reliable.

In some preferred embodiments, the Z-gametologous sequence(s) amplified or bound by oligonucleotides (by amplification primers or probes, respectively, as described in more detail below) of the present invention preferably comprise(s)
- a *hitchcock*-related LTR retroposon insertion sequence or fragment thereof, preferably of the subtype TguLTRSe;
- a CR1 retroposon insertion sequence or fragment thereof, preferably of the subtype CR1-Y2_Aves; and/or
- a CR1 retroposon insertion sequence or fragment thereof, preferably of the subtype CR1-J2_Pass.

Said fragment comprises at least 10, more preferably 15, 20 or 25 consecutive nucleotides of said *hitchcock*-related LTR retroposon insertion sequence or CR1 retroposon insertion sequence.

In some preferred embodiments, the Z-gametologous sequence comprises at least 10, more preferably at least 15, 20 or 25 consecutive nucleotides of the sequence shown in SEQ ID No.7 or 8 (a *hitchcock*-related LTR retroposon insertion sequence)

In some other preferred embodiments, the Z-gametologous sequence comprises at least 10, more preferably at least 15, 20, 25, 30, 35, 40, 45 or 50 consecutive nucleotides of the sequence shown in SEQ ID No.9 (a CR1 retroposon insertion sequence).

More specifically, when the pair of oligonucleotides comprising at least 10 consecutive nucleotides of the sequence shown in SEQ ID No.1 and a second oligonucleotide comprising at least 10 consecutive nucleotides of the sequence shown in SEQ ID No.2 is used as amplification primers, Z-gametologous sequences comprise a *hitchcock*-related LTR retroposon insertion sequence or fragment thereof, preferably of the subtype TguLTR5e.

Likewise, when the pair of oligonucleotides comprising at least 10 consecutive nucleotides of the sequence shown in SEQ ID No.3 and a second oligonucleotide comprising at least 10 consecutive nucleotides of the sequence shown in SEQ ID No.4 is used as amplification primers, Z-gametologous sequences comprise a CR1 retroposon insertion sequence or fragment thereof, preferably of the subtype CR1-Y2_Aves.

Also, when the pair of oligonucleotides comprising at least 10 consecutive nucleotides of the sequence shown in SEQ ID No.5 and a second oligonucleotide comprising at least 10 consecutive nucleotides of the sequence shown in SEQ ID No.6 is used as amplification primers, Z-gametologous sequences comprise a CR1 retroposon insertion sequence or fragment thereof, preferably of the subtype CR1-J2_Pass.

Further, when the pair of oligonucleotides comprising at least 10 consecutive nucleotides of the sequence shown in SEQ ID No.12 and a second oligonucleotide comprising at least 10 consecutive nucleotides of the sequence shown in SEQ ID No.13 is used as amplification primers, Z-gametologous sequences comprise a CR1 retroposon insertion sequence or fragment thereof, preferably of the subtype CR1-J2_Pass.

Said fragment comprises at least 10, more preferably 15, 20 or 25 consecutive nucleotides of said hitchcock-related LTR retroposon insertion sequence or CR1 retroposon insertion sequence.

In a preferred embodiment, said *hitchcock*-related LTR retroposon insertion sequence or fragment is shown in SEQ ID No.7 or 8 and said CR1 retroposon insertion sequence is shown in SEQ ID No.9.

When used herein, the term "oligonucleotide" encompasses "primer(s)" and "probe(s)", but is not limited to oligonucleotides or nucleic acids. The term "oligonucleotide" encompasses molecules that are analogs of nucleotides, as well as nucleotides. Nucleotides and polynucleotides, as used herein shall be generic to polydeoxyribonucleotides (containing 2-deoxy-D-ribose), to polyribonucleotides (containing D-ribose), to any other type of polynucleotide which is an N- or C-glycoside of a purine or pyrimidine base, and to other polymers containing non-nucleotidic backbones, for example, polyamide (e.g., peptide nucleic acids (PNAs)) and polymorpholino (e.g., commercially available from the Anti-Virals, Inc.) and other synthetic sequence-specific nucleic acid polymers providing that the polymers contain nucleobases in a configuration which allows for base pairing and base stacking, such as is found in DNA and RNA.

An "oligonucleotide primer" or "primer" is preferably an oligonucleotide that capable of acting as a point of initiation of synthesis of a primer extension product which is complementary to a nucleic acid strand (template or target sequence) when placed under suitable conditions (e.g., buffer, salt, temperature and pH) in the presence of nucleotides and an agent for nucleic acid polymerization, such as DNA dependent or RNA dependent polymerase (i.e., an amplification primer, as sometimes used herein). A primer must be sufficiently long to prime the synthesis of extension products in the presence of an agent for polymerization. A typical primer contains at least about 10 nucleotides in length of a sequence substantially complementary or homologous to the target sequence, but somewhat longer primers are preferred. For example, primers contain about 15-26 nucleotides but longer primers may also be employed, especially when the primers contain additional sequences such as a promoter sequence for a particular polymerase. A primer binds to or hybridizes with its (complementary) target sequence. Either a primer is fully complementary with its target sequence or substantially complementary. Particularly preferred primers are oligonucleotides comprising at least 10 consecutive nucleotides of any one of the sequences shown in SEQ ID Nos.1-6.

An "oligonucleotide probe", "probe" or "capture probe" is preferably an oligonucleotide that is preferably a labelled segment of a nucleic acid such as DNA or RNA used to find or detect a specific sequence (target sequence) of nucleotides in a nucleic acid molecule. A probe hybridizes to a specific sequence of nucleotides in a nucleic acid molecule comprising a nucleotide sequence (i.e., hybridization probe). A probe can be a heterologous or homologous hybridization probe.

A heterologous probe is a hybridization probe that is substantially complementary to its target sequence, i.e., it is similar to, but not exactly complementary to the target sequence. A homologous probe is a hybridization probe that is fully (exactly) complementary to the target sequence. The probe of the present invention is preferably a heterologous hybridization probe, but may also be a homologous hybridization probe. When used herein, a target sequence is preferably a *hitchcock*-related LTR retroposon or a CR1 retroposon or a fragment of each of them as described herein. A probe to that sequence can, for example, be applied in addition to the oligonucleotide primers of the present invention.

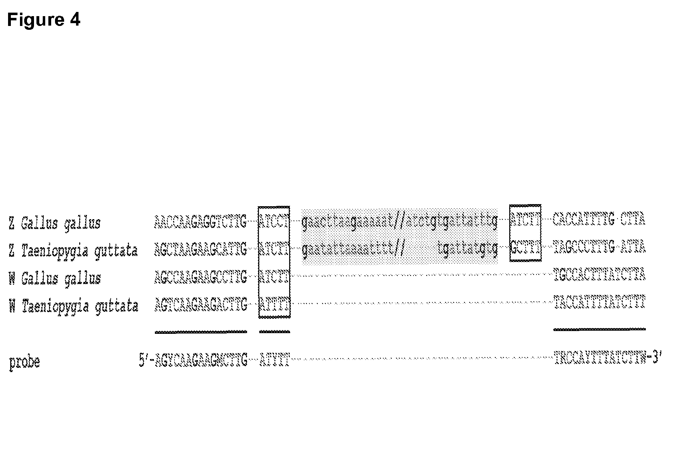

Another preferred target sequence is a W-gametologous sequence which corresponds to that Z-gametologous sequence that is 5' and 3', respectively to the retroposon insertion in intron 9 or 16 of the *CHD1* gene or that is 5' and 3', respectively to the retroposon insertion in intron 16 of the *NIPBL* gene. That preferred target sequence is not present in the Z gametolog in the same nucleotide context as in the W gametolog, since the Z gametolog is "interrupted" by a retroposon insertion in that preferred target sequence (see Figure 4 for an exemplary alignment of *CHD1* intron 16 including an example of a suitable hybridization probe). Thus, the presence of a retroposon in the Z gametolog allows the generation of specific probes that preferably detect the female-specific "empty" retroposon insertion site in the W-gametologous sequence and not the Z gametolog, where the probe's binding site is interrupted due to the retroposon insertion. Exemplary sequences of W-specific hybridization probes for targeting *CHD1* intron 9 (SEQ ID No.14: 5'-GCAATGGATTTATWCAGTTRTGATARTTWTTAA-3'), CHD1 intron 16 (SEQ ID No.15: 5'-AGYCAAGAAGMCTTGATYTTTRCCAYTTTATCTTW-3') or *NIPBL* intron 16 (SEQ ID No.16: 5'-AAYCTGATTSTATTCAAATATCCTTTAATAACTTTA-3') are also given in the figure legend of Figure 4. Each of these W-specific hybridization probes is thus a preferred embodiment of a probe of the present invention. It can be applied alone in the methods or means of the present invention or in combination with the primers as described herein.

The skilled person can readily find such target sequences in the W gametolog by the following method:
(a) identifying Z-gametologous sequences which comprise CR1 retroposon and/or *hitchcock*-related LTR retroposon sequences;
(b) aligning said Z-gametologous sequences identified in (a) with corresponding W-gametologous sequences;
(c) determining whether said aligned W-gametologous sequences lack the CR1 and/or *hitchcock*-related LTR retroposon sequences; and
(d) providing a probe, wherein said probe is substantially complementary to a nucleotide sequence (region) that spans the "empty" retroposon insertion site in the W gametolog.
   Put it differently, said probe comprises upstream (5') and downstream (3') sequences that are adjacent to the retroposon insertion site in the Z gametolog. Said probe does preferably not contain CR1 and/or *hitchcock*-related LTR retroposon sequences.

Step (a) of the probe identification method is preferably performed in that the Z gametolog is screened for retroposons by (i) using RepeatMasker (http://www.repeatmasker.org/cgi-bin/WEBRepeatMasker) or Censor (http://www.girinst.org/censor/index.php) and by (ii) by comparing the Z-gametologous sequences to nucleotide sequences (flexible nucleotide positions are indicated using the IUPAC nucleic acid nomenclature) of CR1 (5'-... RGTTGGACYNGATGAYCYYYNDRGGTCYYTTCCAACYYNNNNN[ATTCTRTG]n-3') (SEQ ID No.9) or *hitchcock*-related LTR (5'-TGTVHTRGKTYYRGCTGRGRTRGAR...YRCYKGCYGGGGTYAAMCCACVACA-3') (SEQ ID No.7 or 8) retroposons.

Preferred Z-gametologous sequences that can be applied in step (a) are intron 9 and/or 16 of the *CHD1* gene and/or intron 16 of the *NIPBL* gene.

Step (b) of the probe identification method is preferably performed by aligning Z-gametologous sequences with corresponding W-gametologous sequences. "Corresponding" when used in the context of the alignment means W-gametologous sequences which are substantially identical and/or fully identical to Z-gametologous sequences, or *vice versa*. In order to determine whether Z- and W-gametologous sequences correspond to each other, the skilled person can use means and methods well-known in the art, e.g., alignments, either manually or by using computer programs such as BLAST2.0, which stands for Basic Local Alignment Search Tool, MAFFT or ClustalW or any other adequate program which is suitable to generate sequence alignments.

Accordingly, in step (c) of the probe identification method the skilled person can then preferably determine whether W-gametologous sequences lack the CR1 and/or *hitchcock*-related LTR retroposon sequences which were identified in the Z-gametologous sequence.

Once identified and designed, said probe can preferably be tested as to whether it is capable of detecting the W gametolog, but not the Z gametolog. A probe identifiable in accordance with the afore-described method for identifying a probe can be applied in the alternative or also in addition to the oligonucleotide primers of the present invention. Preferably, it is applied as such in a method, use or kit of the present invention.

Accordingly, the present invention provides preferably a probe that is substantially complementary to a nucleotide sequence (region) that comprises at least 5, 10, 15, 20, 25, 30, 35, 40, 45 or 50 nucleotides of (i) intron 9 of the CHD1 gene located on the W gametolog, (ii) intron 16 of the CHD1 gene located on the W gametolog or (iii) intron 16 of the *NIPBL* gene located on the W gametolog, wherein said nucleotide sequence spans the region where a retroposon insertion is present in the corresponding intron located on the Z gametolog.
Put it differently, said nucleotide sequence spans the region that "lacks" the retroposon insertion in comparison to the W gametolog; see Figure 4 for an illustration.

In the alternative, other preferred probes are substantially complementary to a nucleotide sequence that
(i) comprises at least 5, 10, 11, 12, 13, 14, 15, 16, 17, 18, 19, 20, 21, 22, 23, 24, or 25 nucleotides of the nucleotide sequence that is 5' to the retroposon insertion site in intron 9 of the *CHD1* gene located on the Z gametolog, intron 16 of the *CHD1* gene located on the Z gametolog, or intron 16 of the *NIPBL* gene located on the Z gametolog; and;
(ii) comprises at least 5, 10, 11, 12, 13, 14, 15, 16, 17, 18, 19, 20, 21, 22, 23, 24, or 25 nucleotides of the nucleotide sequence that is 3' to the retroposon insertion site in intron 9 of the *CHD1* gene located on the Z gametolog, intron 16 of the *CHD1* gene located on the Z gametolog, or intron 16 of the *NIPBL* gene located on the Z gametolog.

"5' to the retroposon insertion site" means that this nucleotide sequence comprises nucleotides that are immediately 5' to the retroposon sequences as described herein, but do not comprise retroposon sequences.

"3' to the retroposon insertion site" means that this nucleotide sequence comprises nucleotides that are immediately 3' to the retroposon sequences as described herein, but do not comprise retroposon sequences.

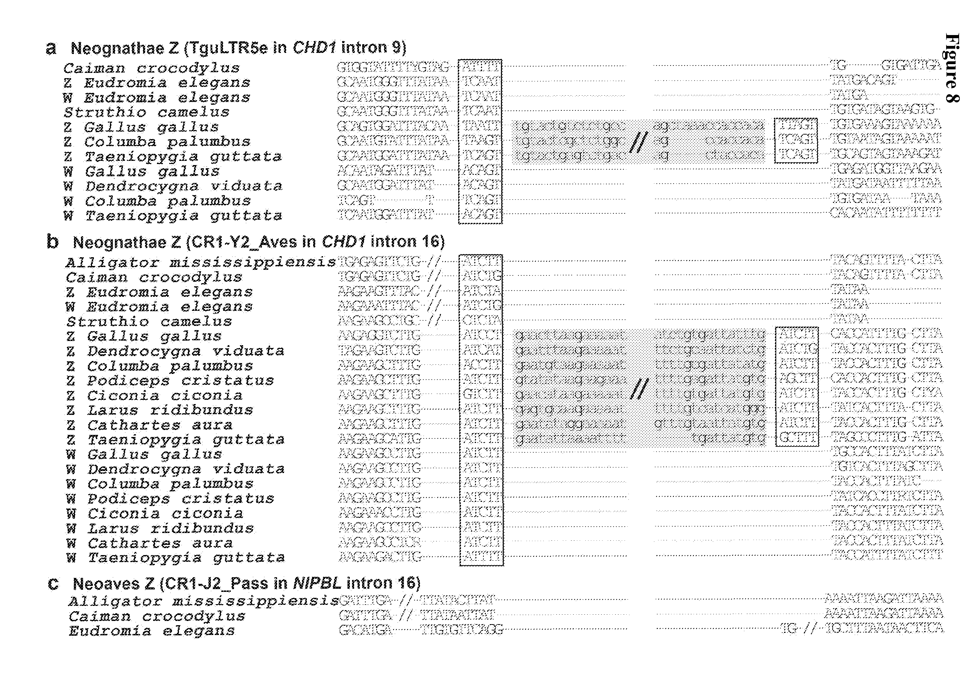

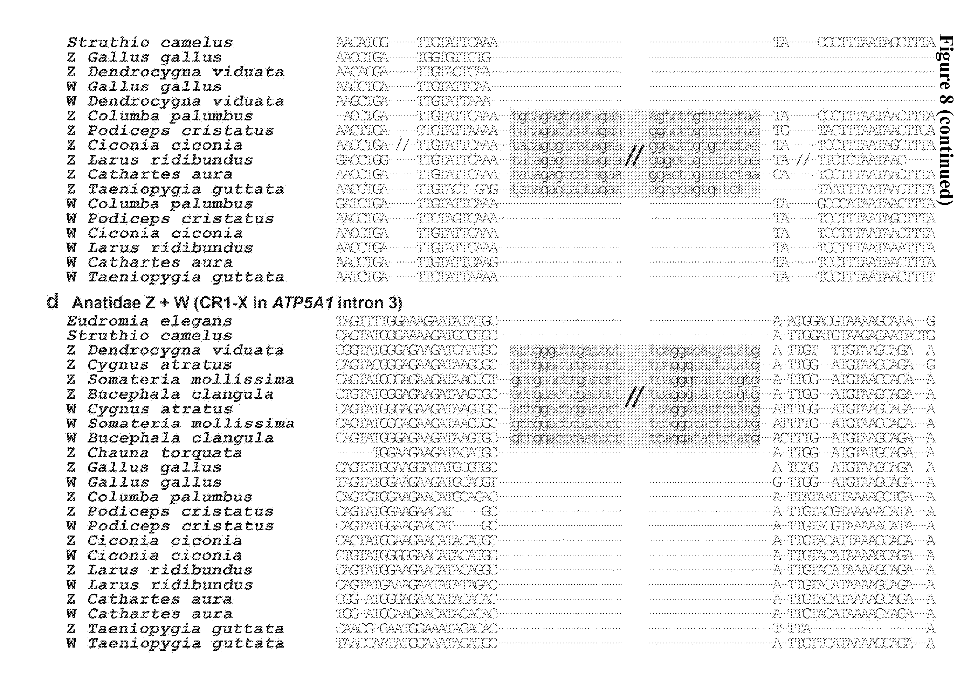

It is essential that, in the case of retroposon insertions flanked by a target site duplication (e.g., 5 bp in hitchcock-related LTR retroposons and <5 bp in some CR1 retroposons; see Figure 8), the singular (unduplicated) target site motif is included in said nucleotide sequence (e.g., the motif "ATYTT" in Figure 4).

The nucleotide sequence of (i) and (ii) may be directly linked (e.g., covalently bonded) to each other or linked by a nucleotide spacer. Said spacer may have length of 1, 2, 3, 4, 5, 6, 7, 8, 9, 10, 11, 12, 13, 14, 15, 20 or more nucleotides and, preferably, does not contain retroposon-derived sequences.

Examples of probes can be derived from the sequences shown in Figure 8. Namely, Figure 8 shows in lower case letters on gray background the 5' and 3' ends of the retroposon insertions. Nucleotide sequences 5' and 3', respectively of said ends can be "fused" (including the singular target site motif, if a target site duplication is flanking the retroposon insertion) and used as target sequence for a probe.

A preferred example of a probe may comprise at least 5, 10, 11, 12, 13, 14, 15, 20, 25 or 30 nucleotides of any one of the sequences shown in SEQ ID Nos.14-16 or a complement thereof.

Less preferred examples of probes are oligonucleotides that are substantially complementary to a nucleotide sequence comprising at least 10 consecutive nucleotides of any one of the sequences shown in SEQ ID Nos.1-6.

Other preferred examples of probes are oligonucleotides that are substantially complementary to a nucleotide sequence comprising at least 10 consecutive nucleotides of
- a *hitchcock*-related LTR retroposon insertion sequence, preferably of the subtype TguLTR5e, or a complement thereof;
- a CR1 retroposon insertion sequence, preferably of the subtype CR1-Y2_Aves, or a complement thereof; or
- a CR1 retroposon insertion sequence, preferably of the subtype CR1-J2_Pass, or a complement thereof.
The skilled person knows that sequences of the named and other subtypes of retroposons are publicly available in Repbase (http://www.girinst.org/repbase/index.html)

By the term "complement thereof" is meant a sequence that is substantially complementary to a target sequence. In that context, the target sequence is a retroposon sequence as described herein.

Further preferred examples of probes are oligonucleotides that are substantially complementary to a nucleotide sequence that comprises at least 10 consecutive nucleotides of the sequence shown in any one of SEQ ID Nos.7-9 or a complement thereof. Other even more preferred examples of probes are oligonucleotides that are substantially complementary to a nucleotide sequence that comprises not more than 50 nucleotides of the sequence shown in any one of SEQ ID Nos.7-9 or a complement thereof. For example, a probe has a length of 10-50 nucleotides, preferably a length of 12-50 nucleotides, more preferably a length of 15-50 nucleotides, even more preferably a length of 20-50 nucleotides.

An oligonucleotide probe of the present invention can preferably be applied in the methods and uses or can be comprised in a kit as described herein.

In some embodiments, the primers and/or probes include oligonucleotides that hybridize to a target nucleic acid sequence over the entire length of the oligonucleotide sequence. Such sequences can be referred to as "fully complementary" with respect to each other. Where an oligonucleotide is referred to as "substantially complementary" with respect to a nucleic acid sequence herein, the two sequences can be fully complementary, or they may form mismatches upon hybridization, but retain the ability to hybridize under stringent conditions or standard PCR conditions as discussed below. As used herein, the term "standard PCR conditions" include, for example, any of the PCR conditions disclosed herein, or known in the art, as described in, for example, PCR: A Practical Approach, M. J. McPherson, P. Quirke, and G. R. Taylor, Eds., 2001, Oxford University Press, Oxford, England, and PCR Protocols: Current Methods and Applications, B. White, Ed., 1993, Humana Press, Totowa, NJ.

As used herein, the term "substantially complementary" refers to the complementarity between two nucleic acids, e.g., the complementary region of the capture probe and the target sequence, and/or between the linker sequence of the capture probe and the complementary region of the competitor nucleic acid. The complementarity need not be perfect; there may be any number of base pair mismatches that between the two nucleic acids. However, if the number of mutations is so great that no hybridization can occur under even the least stringent of hybridization conditions, the sequence is not a substantially complementary sequence. When two sequences are referred to as "substantially complementary" herein, it is meant that the sequences are sufficiently complementary to each other to hybridize under the selected reaction conditions. The relationship of nucleic acid complementarity and stringency of hybridization sufficient to achieve specificity is well known in the art and described further below in reference to sequence identity, melting temperature and hybridization conditions. Therefore, substantially complementary sequences can be used in any of the detection methods described herein. Such probes can be, for example, perfectly complementary or can contain from 1 to many mismatches so long as the hybridization conditions are sufficient to allow, for example discrimination between a target sequence and a non-target sequence. Accordingly, substantially complementary sequences can refer to sequences ranging in percent identity from 100, 99, 98, 97, 96, 95, 94, 93, 92, 91, 90, 89, 85, 80, 75 or less, or any number in between, compared to the reference sequence.

"Stringent conditions" or "high stringency conditions", as defined herein, may be identified by those that: (1) employ low ionic strength and high temperature for washing, for example 0.015 M sodium chloride/0.0015 M sodium citrate/0.1 % sodium dodecyl sulfate at 50°C; (2) employ during hybridization a denaturing agent, such as formamide, for example, 50% (v/v) formamide with 0.1% bovine serum albumin/0.1% Ficoll/0.1% polyvinylpyrrolidone/50mM sodium phosphate buffer at pH 6.5 with 750 mM sodium chloride, 75 mM sodium citrate at 42°C; or (3) employ 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 µg/ml), 0.1% SDS, and 10% dextran sulfate at 42°C, with washes at 42°C in 0.2 x SSC (sodium chloride/sodium citrate) and 50% formamide at 55°C, followed by a high-stringency wash consisting of 0.1 x SSC containing EDTA at 55°C.

"Moderately stringent conditions" may be identified as described by Sambrook et al., Molecular Cloning: A Laboratory Manual, New York: Cold Spring Harbor Press, 2001, and include (exemplarily) the use of washing solution and hybridization conditions (e.g., temperature, ionic strength and %SDS) less stringent that those described above. An example of moderately stringent conditions is overnight incubation at 37°C in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/ml denatured sheared salmon sperm DNA, followed by washing the filters in 1 x SSC at about 37-50°C. The skilled artisan will recognize how to adjust the temperature, ionic strength, etc. as necessary to accommodate factors such as probe length and the like.

The terms "nucleotide" and "polynucleotide" include, for example, 3'-deoxy-2',5'-DNA, oligodeoxyribonucleotide N3'→P5' phosphoramidates, 2'-O-alkyl-substituted RNA, double- and single-stranded DNA, as well as double- and single-stranded RNA, DNA:RNA hybrids, and hybrids between PNAs and DNA or RNA. The terms also include known types of modifications, for example, labels which are known in the art, methylation, "caps," substitution of one or more of the naturally occurring nucleotides with an analog, internucleotide modifications such as, for example, those with uncharged linkages (e.g., methyl phosphonates, phosphotriesters, phosphoramidates, carbamates, etc.), with negatively charged linkages (e.g., phosphorothioates, phosphorodithioates, etc.), and with positively charged linkages (e.g., aminoalklyphosphoramidates, aminoalkylphosphotriesters), those containing pendant moieties, such as, for example, proteins (including nucleases, toxins, antibodies, signal peptides, poly-L-lysine, etc.), those with intercalators (e.g., acridine, psoralen, etc.), those containing chelators (e.g., metals, radioactive metals, boron, oxidative metals, etc.), those containing alkylators, those with modified linkages (e.g., alpha anomeric nucleic acids, etc.), as well as unmodified forms of the polynucleotide or oligonucleotide.

It will be appreciated that, as used herein, the terms "nucleoside" and "nucleotide" will include those moieties which contain not only the known purine and pyrimidine bases, but also other heterocyclic bases which have been modified. Such modifications include methylated purines or pyrimidines, acylated purines or pyrimidines, or other heterocycles. Modified nucleosides or nucleotides will also include modifications on the sugar moiety, e.g., wherein one or more of the hydroxyl groups are replaced with a halogen, an aliphatic group, or are functionalized as ethers, amines, or the like. Other modifications to nucleotides or polynucleotides involve rearranging, appending, substituting for, or otherwise altering functional groups on the purine or pyrimidine base which form hydrogen bonds to a respective complementary pyrimidine or purine. The resultant modified nucleotide or polynucleotide may form a base pair with other such modified nucleotidic units but not with A, T, C, G or U. For example, guanosine (2-amino-6-oxy-9-beta-D-ribofuranosyl-purine) may be modified to form isoguanosine (2-oxy-6-amino-9-beta-D-ribofuranosyl-purine). Such modification results in a nucleoside base which will no longer effectively form a standard base pair with cytosine. However, modification of cytosine (1-β-D-ribofuranosyl-2-oxy-4-amino-pyrimidine) to form isocytosine (1-β-D-ribofuranosyl-2-amino-4-oxy-pyrimidine) results in a modified nucleotide which will not effectively base pair with guanosine but will form a base pair with isoguanosine. Isocytosine is available from Sigma Chemical Co. (St. Louis, Mo.); isocytidine may be prepared by the method described by Switzer et al. (1993) Biochemistry 32:10489-10496 and references cited therein; 2'-deoxy-5-methyl-isocytidine may be prepared by the method of Tor et al. (1993) J. Am. Chem. Soc. 1 15:4461-4467 and references cited therein; and isoguanine nucleotides may be prepared using the method described by Switzer et al. (1993), supra, and Mantsch et al. (1993) Biochem. 14:5593-5601, or by the method described U.S. Pat. No. 5,780,610. The non-natural base pairs referred to as K and π, may be synthesized by the method described in Piccirilli et al. (1990) Nature 343:33-37 for the synthesis of 2,6-diaminopyrimidine and its complement (1-methylpyrazolo[4,3]-pyrimidine-5,7-(4H,6H)-dione. Other such modified nucleotidic units which form unique base pairs have been described, for example, in Leach et al. (1992) J. Am. Chem. Soc. 1 14:3675-3683, or will be apparent to those of ordinary skill in the art.

In some embodiments, the oligonucleotides including primers and probes disclosed herein can contain locked nucleic acids (LNA). "Locked nucleic acids" (LNAs) are ribonucleotides which contain a methylene bridge which joins the 2' oxygen of the ribose with the 4' carbon; see Braasch and Corey (2001), Chem. Biol. 8, 1-7. LNAs are available commercially, for example, from the company Proligo, Boulder, Colo., USA. Phosphorothioates are also known to the person skilled in the art. In some embodiments, the "primers" or "probes" disclosed herein can include 1, 2, 3, 4, 5, 6, 7, 8, 9, 10, or more LNAs.

Preferably, the oligonucleotides, in particular oligonucleotide primers, contain a universal base. As used herein, the term "universal base" refers to a nucleotide analog that can hybridize to more than one nucleotide selected from A, T, C, and G. In some embodiments, the universal base can be selected from the group consisting of deoxyinosine, 3-nitropyrrole, 4-nitroindole, 6-nitroindole, 5-nitroindole. Preferably, the universal base is deoxyinosine. In some embodiments, the set of amplification primers, and probes disclosed herein include at least one primer and/or probe that has 1, 2, 3, 4, 5, 6, 7, 8, 9, 10 or more universal bases.
Primers and/or probes can be provided in any suitable form, included bound to a solid support, liquid, and lyophilized, for example.

In some embodiments, the oligonucleotides, both primers and probes, are labeled with a detectable label. Preferred detectable labels are selected from the group consisting of luminescent markers, fluorescent markers, radioactive markers and enzyme markers.

Labels of interest include directly detectable and indirectly detectable radioactive or nonradioactive labels such as fluorescent dyes. Directly detectable labels are those labels that provide a directly detectable signal without interaction with one or more additional chemical agents. Examples of directly detectable labels include fluorescent labels. Indirectly detectable labels are those labels which interact with one or more additional members to provide a detectable signal. In this latter embodiment, the label is a member of a signal producing system that includes two or more chemical agents that work together to provide the detectable signal. Examples of indirectly detectable labels include biotin or digoxigenin, which can be detected by a suitable antibody coupled to a fluorochrome or enzyme, such as alkaline phosphatase. In many preferred embodiments, the label is a directly detectable label. Directly detectable labels of particular interest include fluorescent labels. Fluorescent labels that find use in the embodiments disclosed herein include a fluorophore moiety. Specific fluorescent dyes of interest include: xanthene dyes, e.g., fluorescein and rhodamine dyes, such as fluorescein isothiocyanate (FITC), 2-[ethylamino)-3-(ethylimino)-2-7-dimethyl-3H-xanthen-9-yl]benzoic acid ethyl ester monohydrochloride (R6G) (emits a response radiation in the wavelength that ranges from about 500 to 560 nm), 1,1',3,3,3',3'-Hexamethylindodicarbocyanine iodide (HIDC) (emits a response radiation in the wavelength that ranged from about 600 to 660 nm), 6-carboxyfluorescein (commonly known by the abbreviations FAM and F), 6-carboxy-2',4',7',4,7-hexachlorofluorescein (HEX), 6-carboxy-4',5'-dichloro-2',7'-dimethoxyfluorescein (JOE or J), N,N,N',N'-tetramethyl-6-carboxyrhodamine (TAMRA or T), 6-carboxy-X-rhodamine (ROX or R), 5-carboxyrhodamine-6G (R6G5 or G5), 6-carboxyrhodamine-6G (R6G6 or G6), and rhodamine 110; cyanine dyes, e.g., Cy3, Cy5 and Cy7 dyes; coumarins, e.g., umbelliferone; benzimide dyes, e.g., Hoechst 33258; phenanthridine dyes, e.g., Texas Red; ethidium dyes; acridine dyes; carbazole dyes; phenoxazine dyes; porphyrin dyes; polymethine dyes, e.g., cyanine dyes such as Cy3 (emits a response radiation in the wavelength that ranges from about 540 to 580 nm), Cy5 (emits a response radiation in the wavelength that ranges from about 640 to 680 nm), etc; BODIPY dyes and quinoline dyes. Specific fluorophores of interest include: Pyrene, Coumarin, Diethylaminocoumarin, FAM, Fluorescein Chlorotriazinyl, Fluorescein, RI 10, Eosin, JOE, R6G, HIDC, Tetramethylrhodamine, TAMRA, Lissamine, ROX, Napthofluorescein, Texas Red, Napthofluorescein, SYBR green, Cy3, and Cy5, and the like.

In preferred embodiments, the oligonucleotide, in particular oligonucleotide probe is a molecular beacon probe, a TAQMAN™ probe, or a SCORPION™ probe. For example, in some embodiments, a probe disclosed herein includes one or more molecular beacon probes.

In some embodiments, the probes, are labeled using an FAM fluorophore quenching molecule and a DABCYL quencher. In some embodiments, the probes are TAQMAN™ probes, molecular beacon probes, or SCORPION™ probes. In some embodiments, the one or more of the amplification primers can be labeled, e.g., with a fluorescent moiety, such as SYBR green, or the like.

In various embodiments, the oligonucleotides disclosed herein can be modified to include additional nucleotides at the 5' or the 3' terminus. Likewise, in some embodiments, the primer and probe sequences can be modified by having nucleotides substituted within the sequence. It is understood that oligonucleotides of the present invention may contain minor deletions, additions and/or substitutions of nucleic acid bases, to the extent that such alterations do not negatively affect the yield or product obtained to a significant degree. Where oligonucleotides according to the present invention are used as probes, the alterations should not result in lowering the hybridization efficiency of the probe.

It is preferably recognized that the primer and probe sequences must contain enough complementarity to hybridize specifically to the respective target nucleic acid sequence. In this manner, at least 1, 2, 3, 4, or up to about 5 nucleotides can be substituted.

In a second aspect, the present invention provides a method for the determination of the sex of an avian subject, comprising
(a) contacting a nucleotide sequence comprised in a sample from said subject with a pair of oligonucleotides, in particular oligonucleotide primers, described herein;
(b) amplifying said nucleotide sequence;
(c) determining the sex of said avian subject by the presence of amplification products of the same size or amplification products of two different sizes,
wherein amplification products of the same size are indicative of a male sex and amplification products of two different sizes are indicative of a female sex.

As mentioned above, the present inventors found that introns 9 and 16 of the *CHD1* gene and intron 16 of the *NIPBL* gene of the Z chromosome contain retroposed elements which are, however, lacking in the respective introns of the W chromosome. Accordingly, when the Z chromosomal region that contains said retroposed element is amplified, the amplification product is larger in size than the W chromosomal region which does not contain said retroposed element (Figure 1). In view of the fact that male avian species have two Z chromosomes, the amplification results in two amplification products of the same size. However, a female avian species has also a W chromosome and, thus, when the W chromosome is amplified that lacks the retroposed element, the amplification product is smaller than that obtained by the amplification of the Z chromosome.

The embodiments disclosed herein can be used to determine the sex of an avian subject in a sample. An "avian subject" includes neognathous birds encompassing the following avian orders *sensu* del Hoyo et al. (cited above): Anseriformes, Apodiformes, Caprimulgiformes, Charadriiformes, Ciconiiformes, Coliiformes, Columbiformes, Coraciiformes, Cuculiformes, Falconiformes, Galbuliformes, Galliformes, Gaviiformes, Gruiformes, Opisthocomiformes, Passeriformes, Pelecaniformes, Piciformes, Phoenicopteriformes, Podicipediformes, Procellariiformes, Psittaciformes, Pterocliformes, Sphenisciformes, Strigiformes, Trogoniformes. As used herein, the term "sample" can refer to a sample from one or any number of sources, including, but not limited to, muscle tissue or bodily fluids from an avian subject such as blood. However, the term "sample" also includes a feather tip or feather pulp. In any event, the sample is intended to comprise a nucleic acid from an avian subject whose sex is to be determined in accordance with the methods and assasy of the present invention.

In some embodiments, the methods and assays described herein can be performed directly on a sample, without further manipulation of the sample. In some embodiments, the sample is manipulated, e.g., processed to extract nucleic acids, or purified, expanded, or otherwise manipulated.

A nucleic acid comprised in a sample from an avian subject is preferably genomic DNA. Genomic DNA is, for example, isolated from blood or muscle tissue of an avian subject using conventional phenol-chloroform extraction, whereas contour feathers can be processed either via the QIAamp DNA Micro kit (Qiagen, Hilden) modified according to Bush (2005): http://www.aviangenetics.com/DNA_Extraction_Protocols/plucked_contour_feathers.html or via rapid simple alkaline extraction following Malagó et al. (2002), BMC Biotechnol. 2:19.

In some embodiments, a sample of an avian subject can be contacted with a pair of oligonucleotides, for example, when said oligonucleotides are applied as amplification primers. In some embodiments, the sample can be contacted with an oligonucleotide, for example, when using an oligonucleotide probe.

The oligonucleotide sequences according to the present invention are especially useful in methods for the amplification of nucleotide sequences. Said nucleotide sequences are preferably obtained or isolated from an avian subject. For example, a feather tip or feather pulp from an avian subject comprises a nucleic acid comprising a nucleotide sequence that can be applied in the methods of the present invention for the determination of the sex of an avian subject.

In some preferred embodiments, the nucleotide sequence is amplified by an amplification technique selected from the group consisting of PCR, nested PCR, multiplex PCR, ligase chain reaction (LCR) , transcription-based amplification systems (TAS), self-sustained sequence replication (SSR), nucleic acid sequence-based amplification (NASBA), strand displacement amplification (SDA) or branched DNA (bDNA). Yet, PCR is a particularly preferred amplification technique. Figure 5 provides an overview on the fast and convenient application of PCR. Notably, the analysis of the PCR amplification reaction can conveniently be analyzed by simple agarose gel electrophoresis (e.g., 1% agarose and Tris-acetate-EDTA buffer, TAE).

In some other preferred embodiments, in the amplification step of the method of the present invention a probe (as described herein above) is present. Said probe can either be a homologous or heterologous probe as described herein. It can either be fully complementary or substantially complementary as also described herein.

In some embodiments, binding or annealing of the primers and/or probes to target nucleic acid sequences is accomplished through hybridization. It will be appreciated by one skilled in the art that specific hybridization is achieved by selecting sequences which are at least substantially complementary to the target or reference nucleic acid sequence. This includes base-pairing of the oligonucleotide target nucleic acid sequence over the entire length of the oligonucleotide sequence. Such sequences can be referred to as "fully complementary" with respect to each other. Where an oligonucleotide is referred to as "substantially complementary" with respect to a nucleic acid sequence herein, the two sequences can be fully complementary, or they may form mismatches upon hybridization, but retain the ability to hybridize under stringent conditions or standard PCR conditions as discussed below.

In some embodiments, the sample is contacted with a set of amplification primers and a probe. Preferably, the amplification primers and probes hybridize to target nucleic acids under a single set of conditions, i.e., stringent conditions, including standard PCR conditions discussed below. "Stringent conditions" are defined herein above. Stringent hybridization conditions can vary (for example from salt concentrations of less than about 1 M, more usually less than about 500 mM and preferably less than about 200 mM) and hybridization temperatures can range, for example, from as low as 0°C to greater than 22°C, greater than about 30°C and (most often) in excess of about 37°C depending upon the lengths and/or the nucleic acid composition of the probes. Longer fragments may require higher hybridization temperatures for specific hybridization. As several factors affect the stringency of hybridization, the combination of parameters is more important than the absolute measure of a single factor. Accordingly, by way of example, the term "stringent hybridization conditions" can refer to either or both of the following: a) 6 x SSC at about 45°C, followed by one or more washes in 0.2 x SSC, 0.1 % SDS at 65°C, and b) 400 mM NaCl, 40 mM PIPES pH 6.4, 1 mM EDTA, 50°C or 70°C for 12-16 hours, followed by washing. In some embodiments, the term "stringent conditions" can refer to standard PCR conditions.

In some embodiments, the sample is contacted with a set of amplification primers under standard PCR conditions. For a review of PCR technology, including standard PCR conditions, see DNA Methods in Clinical Microbiology, Singleton, P., published by Dordrecht; Boston: Kluwer Academic, 2000, Molecular Cloning to Genetic Engineering White, B. A., Ed., in Methods in Molecular Biology 67: Humana Press, Totowa, 1997, and "PCR Methods and Applications", Cold Spring Harbor Laboratory Press, 1991-1995. Non-limiting examples of "PCR conditions" include the conditions disclosed in the references cited herein, such as, for example, 50 mM KCI, 10 mM Tris-HCl (pH 9.0), 0.1% Triton X-100, 2.5 mM MgCl₂, with an annealing temperature of 72°C; or 4 mM MgCl₂, 10 mM Tris, pH 8.3, 10 mM KCI, 5 mM (NH₄)₂SO₄, 0.15 mg BSA, 4% Trehalose, with an annealing temperature of 59°C, or 50 mM KCI, 10 mM Tris-HCl(pH 9.0), 0.1% Triton X-100, 2.5 mM MgCl₂, with an annealing temperature of 55°C, or the like. A particularly preferred PCR protocol is described in the Examples and in Figure 5. This protocol is particularly preferred with the primers shown in any one of SEQ ID Nos.1-6.

Generally, in PCR, a target polynucleotide sequence is amplified by reaction with at least one oligonucleotide primer or pair of oligonucleotide primers. The primer(s) hybridize to a complementary region of the target nucleic acid and a nucleic acid polymerase extends the primer(s) to amplify the target sequence. Under conditions sufficient to provide polymerase-based nucleic acid amplification products, a nucleic acid fragment of one size dominates the reaction products (the target polynucleotide sequence that is the amplification product). The amplification cycle is repeated to increase the concentration of the single target polynucleotide sequence. The reaction can be performed in any thermocycler commonly used for PCR. However, preferred are cyclers with realtime fluorescence measurement capabilities, for example, SMARTCYCLER® (Cepheid, Sunnyvale, CA), ABI PRISM 7700® (Applied Biosystems, Foster City, CA), ROTOR-GENE™; (Corbett Research, Sydney, Australia), LIGHTCYCLER® (Roche Diagnostics Corp, Indianapolis, IN), ICYCLER® (Biorad Laboratories, Hercules, CA) and MX4000® (Stratagene, La Jolla, CA).
In some embodiments, a labeled probe can be used to detect the extension product generated by PCR amplification. Any probe format utilizing a labeled probe comprising sequences disclosed herein can be used, e.g., SCORPION™ probes, sunrise probes, TAQMAN™ probes, or molecular beacon probes as is known in the art or described elsewhere herein. In some embodiments, the probes can be used at a concentration of about 0.01 µM, 0.02 µM, 0.03 µM, 0.04 µM, 0.05 µM, 0.06 µM, 0.07 µM, 0.08 µM, 0.09 µM, 0.1 µM, 0.1 1 µM, 0.12 µM, 0.13 µM, 0.14 µM, 0.15 µM, 0.16 µM, 0.17 µM, 0.18 µM, 0.19 µM, 0.2 µM, 0.21 µM, 0.22 µM, 0.23 µM, 0.24 µM, 0.25 µM, 0.26 µM, 0.27 µM, 0.28 µM, 0.29 µM, 0.3 µM, 0.31 µM, 0.32 µM, 0.33 µM, 0.34 µM, 0.35 µM, 0.36 µM, 0.37 µM, 0.38 µM, 0.39 µM, 0.4 µM, 0.42 µM, 0.46 µM, 0.48 µM, 0.5 µM, or more, or any concentration in between.

Methods for setting up a PCR reaction are well known to those skilled in the art. The reaction mixture minimally comprises template nucleic acid (except in the case of a negative control as described below) and oligonucleotide primers and/or probes in combination with suitable buffers, salts, and the like, and an appropriate concentration of a nucleic acid polymerase. As used herein, "nucleic acid polymerase" refers to an enzyme that catalyzes the polymerization of nucleoside triphosphates. Generally, the enzyme will initiate synthesis at the 3'-end of the primer annealed to the target sequence, and will proceed in the 5'-direction along the template until synthesis terminates. An appropriate concentration includes one that catalyzes this reaction in the presently described methods. Known DNA polymerases useful in the methods disclosed herein include, for example, *E. coli* DNA polymerase I, T7 DNA polymerase, *Thermus thermophilus* (Tth) DNA polymerase, *Bacillus stearothermophilus* DNA polymerase, *Thermococcus litoralis* DNA polymerase, *Thermus aquaticus* (Taq) DNA polymerase and *Pyrococcus furiosus* (Pfu) DNA polymerase.

In addition to the above components, the reaction mixture of the present methods includes primers, probes, and deoxyribonucleoside triphosphates (dNTPs).
Usually the reaction mixture will further comprise four different types of dNTPs corresponding to the four naturally occurring nucleoside bases, i.e., dATP, dTTP, dCTP, and dGTP. In some of the embodiments disclosed herein, each dNTP will typically be present in an amount ranging from about 10 to 5000 µM, usually from about 20 to 1000 µM, about 100 to 800 uM, or about 300 to 600 µM.

The reaction mixture applied in the methods of the embodiments disclosed herein further includes an aqueous buffer medium that includes a source of monovalent ions, a source of divalent cations, and a buffering agent. Any convenient source of monovalent ions, such as potassium chloride, potassium acetate, ammonium acetate, potassium glutamate, ammonium chloride, ammonium sulfate, and the like may be employed. The divalent cation may be magnesium, manganese, zinc, and the like, where the cation will typically be magnesium. Any convenient source of magnesium cation may be employed, including magnesium chloride, magnesium acetate, and the like. The amount of magnesium present in the buffer may range from 0.5 to 10 mM, and can range from about 1 to about 6 mM, or about 3 to about 5 mM. Representative buffering agents or salts that may be present in the buffer include Tris, Tricine, HEPES, MOPS, and the like, where the amount of buffering agent will typically range from about 5 to 150 mM, usually from about 10 to 100 mM, and more usually from about 20 to 50 mM, where in certain preferred embodiments the buffering agent will be present in an amount sufficient to provide a pH ranging from about 6.0 to 9.5, for example, about pH 6.0, 6.5, 7.0, 7.5, 8.0, 8.5, 9.0, or 9.5. Other agents that may be present in the buffer medium include chelating agents, such as EDTA, EGTA, and the like. In some embodiments, the reaction mixture can include BSA, or the like.

In preparing the reaction mixture, the various constituent components may be combined in any convenient order. For example, the buffer may be combined with primer, polymerase, and then template nucleic acid, or all of the various constituent components may be combined at the same time to produce the reaction mixture. Alternatively, commercially available premixed reagents can be utilized in the methods disclosed herein according to the manufacturer's instructions, or modified to improve reaction conditions (e.g., modification of buffer concentration, cation concentration, or dNTP concentration, as necessary), including, for example, TAQMAN® Universal PCR Master Mix (Applied Biosystems), OMNIMIX® or SMARTMIX® (Cepheid); Supermix (Bio-Rad Laboratories), LIGHTCYCLER® FastStart (Roche Applied Science, Indianapolis, IN).

Following preparation of the reaction mixture, the reaction mixture can be subjected to primer extension reaction conditions ("conditions sufficient to provide polymerase-based nucleic acid amplification products"), i.e., conditions that permit for polymerase-mediated primer extension by addition of nucleotides to the end of the primer molecule using the template strand as a template. In many embodiments, the primer extension reaction conditions are amplification conditions, which conditions include a plurality of reaction cycles, where each reaction cycle comprises: (1) a denaturation step, (2) an annealing step, and (3) a polymerization step. The number of reaction cycles will vary depending on the application being performed, but will usually be at least 15, more usually at least 20, and may be as high as 60 or higher, where the number of different cycles will typically range from about 20 to 40. For methods where more than about 25, usually more than about 30 cycles are performed, it may be convenient or desirable to introduce additional polymerase into the reaction mixture such that conditions suitable for enzymatic primer extension are maintained.

The denaturation step comprises heating the reaction mixture to an elevated temperature and maintaining the mixture at the elevated temperature for a period of time sufficient for any double-stranded or hybridized nucleic acid present in the reaction mixture to dissociate. For denaturation, the temperature of the reaction mixture will usually be raised to, and maintained at, a temperature ranging from about 85 to 100°C, usually from about 90 to 98°C, and more usually from about 93 to 96°C, for a period of time ranging from about 3 to 120 sec, usually from about 3 sec.

Following denaturation, the reaction mixture will be subjected to conditions sufficient for primer annealing to template nucleic acid present in the mixture (if present), and for polymerization of nucleotides to the primer ends in a manner such that the primer is extended in a 5' to 3' direction using the nucleic acid to which it is hybridized as a template, i.e., conditions sufficient for enzymatic production of primer extension product. In this embodiment, the annealing and extension processes occur in the same step. The temperature to which the reaction mixture is lowered to achieve these conditions will usually be chosen to provide optimal efficiency and specificity, and will generally range from about 50 to 75°C, usually from about 55 to 70°C, and more usually from about 60 to 68°C, more particularly around 60°C. Annealing conditions will be maintained for a period of time ranging from about 15 sec to 30 min, usually from about 20 sec to 5 min, or about 30 sec to 1 minute, or about 30 seconds.

This step can optionally comprise one of each of an annealing step and an extension step with variation and optimization of the temperature and length of time for each step. In a two-step annealing and extension, the annealing step is allowed to proceed as above. Following annealing of primer to template nucleic acid, the reaction mixture will be further subjected to conditions sufficient to provide for polymerization of nucleotides to the primer ends as above. To achieve polymerization conditions, the temperature of the reaction mixture will typically be raised to or maintained at a temperature ranging from about 65 to 75°C, usually from about 67 to 73°C and maintained for a period of time ranging from about 15 sec to 20 min, usually from about 30 sec to 5 min.

In some embodiments, the cycling can include a 15-minute initial denaturation at 95°C, which is performed only once, followed by a denaturation step at 95°C for 1 second, and an annealing/elongation step at 60°C for 25 seconds. This two-step cycle can be repeated multiple times, e.g., about 45 times. In some embodiments, a final elongation step can be added at 72°C for 10 minutes.

In some embodiments, the cycling can include a 15-minute initial denaturation step at 95°C, is followed by multiple cycles (e.g., about 45 cycles) of: denaturation at 95°C for 1 second, annealing at 60°C for 9 seconds and elongation at 72°C for 9 seconds. A final elongation step can be added of 72°C for 10 minutes.

The above cycles of denaturation, annealing, and polymerization may be performed using an automated device, typically known as a thermal cycler. Thermal cyclers that may be employed are described elsewhere herein as well as in U.S. Patents 5,612,473; 5,602,756; 5,538,871; and 5,475,610; the disclosures of which are herein incorporated by reference.

The methods disclosed herein can also be used in non-PCR based applications to detect a target nucleic acid sequence, where such target may be immobilized on a solid support. Methods of immobilizing a nucleic acid sequence on a solid support are known in the art and are described in Ausubel et al, eds. (1995) Current Protocols in Molecular Biology (Greene Publishing and Wiley-Interscience, NY), and in protocols provided by the manufacturers, e.g., for membranes: Pall Corporation, Schleicher & Amp, Schuell; for magnetic beads: Dynal; for culture plates: Costar, Nalgenunc; for bead array platforms: Luminex and Becton Dickinson; and, for other supports useful in the embodiments disclosed herein: CPG, Inc.

The person skilled in the art of nucleic acid amplification knows the existence of other rapid amplification procedures such as ligase chain reaction (LCR), transcription-based amplification systems (TAS), self-sustained sequence replication (3SR), nucleic acid sequence-based amplification (NASBA), strand displacement amplification (SDA) and branched DNA (bDNA) (Persing et al. (1993) Diagnostic Molecular Microbiology: Principles and Applications (American Society for Microbiology, Washington, DC)). The scope of the embodiments disclosed herein is not limited to the use of amplification by PCR, but rather includes the use of any rapid nucleic acid amplification methods or any other procedures that may be useful with the sequences of the embodiments disclosed herein.

In some embodiments, the amplification reaction such as PCR disclosed herein can contain various controls. Such controls can include a "no template" negative control, in which primers, buffer, enzyme(s) and other necessary reagents (e.g., magnesium chloride, nucleotides) are cycled in the absence of added test sample. A positive control including a known target nucleic acid can also be run in parallel. In some embodiments, both a positive control and negative control can be included in the amplification reaction. A single reaction may contain either a positive control, a negative control, or a sample template, or a single reaction may contain both a sample template and a positive control. In addition to "no template" controls, negative controls can also include amplification reactions with non-specific target nucleic acid included in the reaction, or can be samples prepared using any or all steps of the sample preparation (from nucleic acid extraction to amplification preparation) without the addition of a test sample.

Preferably, PCR controls should be performed at the same time as the test sample, using the same reagents, in the same amplification reaction.

Alternatively, the amplification products can be detected using dot blot analysis. Dot blot analysis involves adhering an oligonucleotide probe (such as the one described previously) to a nitrocellulose or solid support such as, but not limited to, a bead (such as, but not limited to, polystyrene beads, magnetic beads, or non-magnetic beads, etc.), walls of a reaction tray, strips (such as, but not limited to, nitrocellulose strips), a test tube. The sample containing the labeled amplification product is added, reacted, washed to remove unbound sample, and a labeled, amplified product attached to the probe is visualized using routine techniques known in the art. A more stringent way to verify the primer extension product or amplification product is through direct sequencing using techniques well known in the art.

In a third aspect, the present invention provides a use of a pair of oligonucleotides as described herein for the determination of the sex of an avian subject. The same embodiments as described in the context of oligonucleotides and methods are applicable for said third aspect, *mutatis mutandis.*

In a fourth aspect, the present invention provides a method for the identification of a pair of oligonucleotides for sexing of an avian subject, comprising the following steps:
(a) identifying Z-gametologous sequences which comprise CR1 retroposon and/or hitchcock-related LTR retroposon sequences;
(b) aligning said Z-gametologous sequences identified in (a) with corresponding W-gametologous sequences;
(c) determining whether said aligned W-gametologous sequences lack the CR1 and/or hitchcock-related LTR retroposon sequences; and
(d) providing a pair of oligonucleotides, wherein each oligonucleotide is substantially complementary to a region that is 5' and 3', respectively, of said aligned sequences and which is capable of simultaneously amplifying both a W-gametologous sequence and a Z-gametologous sequence, the latter comprising CR1 retroposon and/or hitchcock-related LTR retroposon sequence.

Step (a) is preferably performed in that the Z gametolog is screened for retroposons by (i) using RepeatMasker (http://www.repeatmasker.org/cgi-bin/WEBRepeatMasker) or Censor (http://www.girinst.org/censor/index.php) and by (ii) by comparing the Z-gametologous sequences to sequences of CR1 (SEQ ID No.9) or hitchcock-related LTR (SEQ ID No.7 or 8) retroposons
Preferred Z-gametologous sequences that can be applied in step (a) are intron 9 and/or 16 of the *CHD1* gene and/or intron 16 of the *NIPBL* gene.

Step (b) is preferably performed by aligning Z-gametologous sequences with corresponding W-gametologous sequences. "Corresponding" when used in the context of the alignment means W-gametologous sequences which are substantially identical and/or fully identical to Z-gametologous sequences, or *vice versa*. In order to determine whether Z- and W-gametologous sequences correspond to each other, the skilled person can use means and methods well-known in the art, e.g., alignments, either manually or by using computer programs such as BLAST2.0, which stands for Basic Local Alignment Search Tool, MAFFT or ClustalW or any other adequate program which is suitable to generate sequence alignments.

Accordingly, in step (c) the skilled person can then preferably determine whether W-gametologous sequences lack the CR1 and/or *hitchcock*-related LTR retroposon sequences which were identified in the Z-gametologous sequence.

Step (d) is preferably performed in that oligonucleotide primers are designed, wherein each oligonucleotide is substantially complementary to a region that is 5' and 3', respectively, of the aligned sequences (see step (b)) and which is capable of amplifying a Z-gametologous sequence comprising CR1 retroposon and/or *hitchcock*-related LTR retroposon sequence. Similarly, said oligonucleotide is capable of amplifying the corresponding W-gametologous sequence which lacks the CR1 retroposon and/or *hitchcock*-related LTR retroposon sequence. Preferably, said oligonucleotides are located in upstream and downstream exons of intron 9 and/or 16 of the *CHD1* gene and/or in upstream and downstream exons of intron 16 of the *NIPBL* gene.

In a preferred embodiment, said method further comprises step (e) determining whether said pair of oligonucleotides is capable of amplifying a W-gametologous sequence from one or more species selected from the group consisting of *Chloebia gouldiae, Locustella ochotensis*, *Parus major, Parus palustris* and *Taeniopygia guttata*.

When preforming the above described method, oligonucleotides or pairs of oligonucleotides can be identified which are useful for the determination of the sex of an avian subject. Accordingly, in a further aspect the present invention provides oligonucleotides and/or pair of oligonucleotides for sexing of an avian subject obtainable by the above described method.

In a fifth aspect, the present invention also provides "kits". These kits are for the determination of the sex of an avian subject and comprise at least an oligonucleotide or a pair of oligonucleotides as described herein.

In a sixth aspect, the present invention relates to the use of an oligonucleotide or a pair of oligonucleotides described herein for the preparation of a kit for the determination of the sex of an avian subject.

In some preferred embodiments, the kits further comprise an oligonucleotide probe as described herein. Said oligonucleotide probe is further described herein above.

In some further preferred embodiments, the kits further comprise means for performing an amplification technique such as nucleic acid polymerases, nucleotides, buffers, and the like.

Such a kit can comprise a carrier being compartmentalized to receive in close confinement therein one or more containers, such as tubes or vials. One of the containers may contain at least one unlabeled or detectably labeled primer or probe disclosed herein. The primer or primers can be present in lyophilized form or in an appropriate buffer as necessary. One or more containers may contain one or more enzymes or reagents to be utilized in nucleic acid amplification reactions such as PCR reactions. These enzymes may be present by themselves or in admixtures, in lyophilized form or in appropriate buffers.

Finally, the kit can include all of the additional elements necessary to carry out the methods disclosed herein, such as buffers, extraction reagents, enzymes, pipettes, plates, nucleic acids, nucleoside triphosphates, filter paper, gel materials, transfer materials, autoradiography supplies, and the like.

In some embodiments, the kits include additional reagents that are required for or convenient and/or desirable to include in the reaction mixture prepared during the methods disclosed herein, where such reagents include: one or more polymerases; an aqueous buffer medium (either prepared or present in its constituent components, where one or more of the components may be premixed or all of the components may be separate), and the like. The various reagent components of the kits may be present in separate containers, or may all be pre-combined into a reagent mixture for combination with template nucleic acid.

In addition to the above components, in some embodiments, the kits can also include instructions for practicing the methods disclosed herein. These instructions can be present in the kits in a variety of forms, one or more of which may be present in the kit. One form in which these instructions can be present is as printed information on a suitable medium or substrate, e.g., a piece or pieces of paper on which the information is printed, in the packaging of the kit, in a package insert, etc. Yet another means would be a computer readable medium, e.g., diskette, CD, etc., on which the information has been recorded. Yet another means that may be present is a website address that may be used via the internet to access the information at a removed site. Any convenient means may be present in the kits.

### FIGURES

**Figure 1****: Molecular sexing of birds using insertions of retroposed elements (RE).**
In male birds (ZZ), only the larger Z gametolog (containing the RE) is present, whereas female birds (ZW) also exhibit the smaller W gametolog (lacking the RE). Short arrows indicate primer positions for simultaneous PCR amplification of both gametologs of a given intron. Large grey boxes are exons and white boxes are retroposon insertions.

**Figure 2****: Comparison of the three retroposon sexing loci of the present invention.**
The 1% agarose gel photo includes a pUC8 size marker (L) and shows PCR amplicons of a male and a female *Taeniopygia guttata.* In cases of two distinct bands, the lower band represents the W amplicon and the upper band the larger Z amplicon containing a retroposon insertion. The PCR amplicons were amplified using SEQ ID No.1+2 *(CHD1* intron 9), SEQ ID No.3+4 *(CHD1* intron 16) or SEQ ID No.5+6 *(NIPBL* intron 16).

**Figure 3****: Comparison of molecular sexing efficiency.**
*CHD1* intron 16 was amplified using primers of prior art (Fridolfsson & Ellegren, cited herein) or primers of the present invention. Primers of the present invention reliably identify the sex of problematic species where other primers lead to misidentifications. Loc: *Locustella ochotensis,* Pma: *Parus major,* Ppa: *Parus palustris,* Tgu: *Taeniopygia guttata.*

**Figure 4****: Exemplary design of an oligonucleotide probe that exclusively hybridizes to the female-specific W intron of a gametologous intron pair.**
In the given example of *CHD1* intron 16, a probe containing the nucleotide sequence 5'-AGYCAAGAAGMCTTGATYTTTRCCAYTTTATCTTW-3' (SEQ ID No.15) detects the W but not the Z gametolog, because the Z chromosomal binding site (black horizontal line) is interrupted by a retroposon insertion (lowercase letters on grey background) plus duplicated target site (black boxes; such direct repeats or target site duplications are present in the case of LTR retroposon insertions and some CR1 retroposon insertions). Likewise, a probe containing the nucleotide sequence 5'-GCAATGGATTTATWCAGTTRTGATARTTWTTAA-3' (SEQ ID No.14) hybridizes to the W-specific sequence of *CHD1* intron 9 and 5'-AAYCTGATTSTATTCAAATATCCTTTAATAACTTTA-3' (SEQ ID No.16) detects the W gametolog of *NIPBL* intron 16 (alignments of these probes are not shown, but can be reproduced by comparison to Figure 8).

**Figure 5****: Standard operation procedure for molecular sexing of birds using gametologous retroposon insertion loci.**
Overview on an exemplary procedure of how to practice the present invention. Due to space limitations, only primers and agarose gel pictures of *CHD1* intron 16 and *NIPBL* intron 16 (SEQ ID Nos.3-6) are shown. Nevertheless, this procedure is applicable to *CHD1* intron 9 (SEQ ID No.1+2) as well.

**Figure 6****: Hypothetical scenario of the evolution of a gametologous gene pair indicated by insertions of retroposed elements (RE).**
Large grey boxes are exons, small grey boxes are untranslated regions, and white boxes are retroposon insertions. Subsequent to frequent recombination (left), cessation of recombination leads to divergence (right) of the two gametologs. Asterisks denote REs that inserted prior to differentiation; REs marked with a circle were inserted after cessation of recombination. Short arrows indicate primer positions for simultaneous PCR amplification of both gametologs of a given intron.

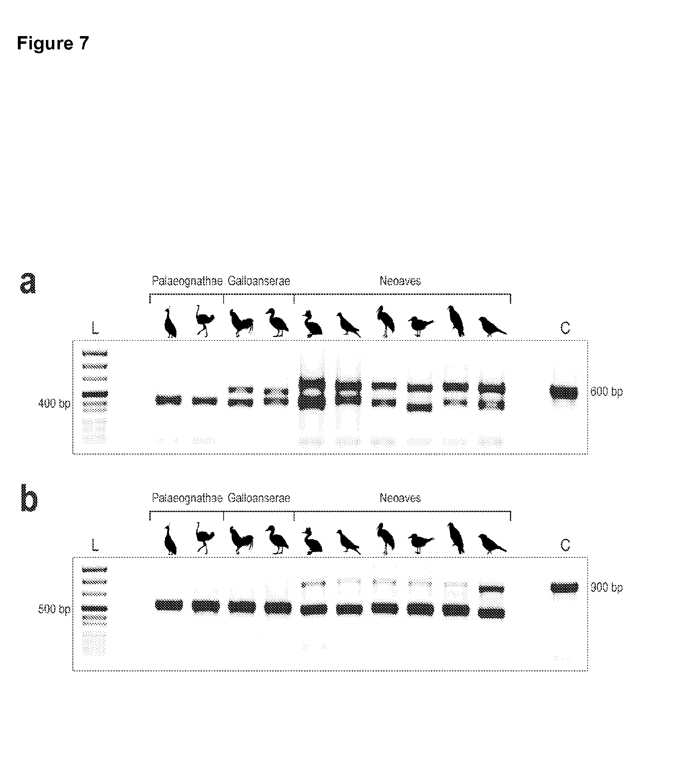

**Figure 7****: PCR products of (a) the *CHD1* intron 16 and (b) the *NIPBL* intron 16 in female birds of all major avian clades.**
Primers are SEQ ID No.3+4 *(CHD1* intron 16) and SEQ ID No.5+6 *(NIPBL* intron 16). Each inverted 1% agarose gel photo includes a pUC8 size marker (L) and a male zebra finch Z amplicon for control (C). In cases of two visible bands, the lower band represents the W amplicon and the upper band the larger Z amplicon containing a retroposon insertion. In earlier branching species only one band is visible, as the Z and W amplicons show no distinct size differences. All avian samples are female and listed in the order of the corresponding silhouettes: Palaeognathae: *Eudromia elegans* (Tinamiformes) and *Struthio camelus* (Struthioniformes), Galloanserae: *Gallus gallus* (Galliformes)and *Dendrocygna viduata* (Anseriformes), Neoaves: *Podiceps cristatus* (Podicipediformes), *Columba palumbus* (Columbiformes), *Ciconia ciconia* (Ciconiiformes), *Larus ridibundus* (Charadriiformes), *Cathartes aura* (Falconiformes) and *Taeniopygia guttata* (Passeriformes).

**Figure 8****: Alignments of the presence/absence regions of the four gametologous intron pairs.**
Potential direct repeats are boxed, 5' and 3' ends of the retroposon insertions are shown in lower case letters on gray background.

### EXAMPLES

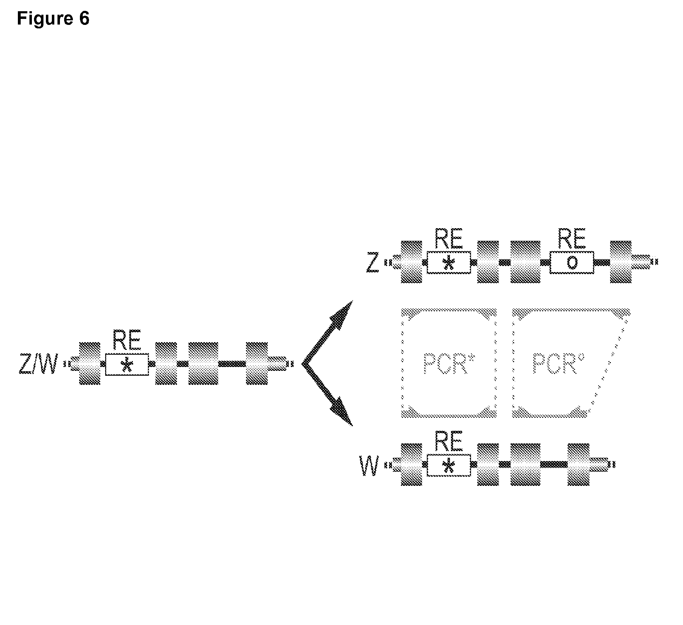

In the case of gametologous gene pairs (Figure 6), it was assumed that a retroposon insertion could be present either in both gametologs of a given genome and absent in the outgroup's gametolog pairs, or present in only one of the gametologs and absent in the other. Accordingly, the following computational procedure was performed.

Computational procedures
Initially, the chicken Z and W chromosomes and the zebra finch Z chromosome (Genome Browser, http://genome.ucsc.edu/cgi-bin/hgBlat), later also all available gametolog sequences in GenBank (http://www.ncbi.nlm.nih.gov/Genbank/) were scanned for retroposed elements using RepeatMasker (http://www.repeatmasker.org/cgi-bin/WEBRepeatMasker) and CENSOR (http://www.girinst.org/censor/index.php). For introns (smaller than 1000 bp) present on both sex chromosomes, the chicken Z, chicken W, zebra finch Z and other available homologous sequences were aligned using MAFFT (E-INS-I, version 6, http://mafft.cbrc.jp/alignment/server/) and inspected by eye. The remaining six feasible cases of either Z-presence/W-presence or Z-presence/W-absence were used for exonic primer design.
To this end, four phylogenetically informative gametologous loci were experimentally analyzed and amplified in representatives of all major avian taxa *sensu* (Hackett et al. (2008), Science 320:1763-1768). All sequences of each locus were aligned, screened for rare genomic changes such as retroposon insertions (Figure 8) and random indels, and subjected to sequence analyses using RAxML (Stamatakis et al. (2008), Syst. Biol. 75:758-771).
W- or Z-linkage of sequenced gametologs was assigned by comparison to the sequence amplified in a conspecific or closely related male. In the case of *ATP5A1,* where both gametologs have about the same size, 10 clones per sample species were sequenced to ensure that both gametologs were obtained. Merely for *Chauna torquata, Columba palumbus* and *Dendrocygna viduata,* only the Z gametolog could be found, respectively, possibly due to a deletion of *ATP5A1* from the W chromosome as observed in a parrot species (De Kloet (2001), J. Mol. Evol. 53:135-143). In all sample species that yielded only one sequence from multiple sequenced clones, the PCR product was subsequently sequenced directly to ensure that only one gametolog was present or amplifiable. To complete our sampling, chicken and zebra finch sequences from Genome Browser and several sequences published in GenBank (AF525979, AF525980, AF165968, AF165979) were added. To ensure correct interpretation of retroposon insertions and random indels, all sequences of each locus were first automatically aligned using MAFFT and then manually realigned, respectively. Phylogenetic analyses of the intronic sequence alignments excluding retroposon insertions were each performed using RAxML with 100 bootstrap replicates.

Taxon sampling
The experimental taxon sampling comprises the following species. Crocodylia: *Alligator mississippiensis* and *Caiman crocodilus,* Palaeognathae: *Eudromia elegans* and *Struthio camelus,* Galloanserae: *Gallus gallus* and *Dendrocygna viduata,* Neoaves: *Podiceps cristatus, Columba palumbus, Ciconia ciconia, Larus ridibundus, Cathartes aura* and *Taeniopygia guttata.* All avian samples are female and listed in the order of the corresponding silhouettes of Figure 7. For *ATP5A1,* a female of the anseriform *Chauna torquata* was added to the sampling.

Primer design/Primer sequences
To PCR-amplify the four loci *(CHD1* introns 9 and 16, *NIPBL* intron 16 and ATP5A1 intron 3) investigated here, the following primer pairs that perform well at an annealing temperature of 52°C were used:
CHD1-i9F (5'-CAGCAGAAATCAATCCAAGAC-3') (SEQ ID No.1)
CHD1-i9R (5'-CAGCCCATTTAACTGATAATCTC-3') (SEQ ID No.2)

CHD1-i16F (5'-GTCCTGATTTTCTCACAGATGG-3') (SEQ ID No.3)
CHD1-i16R (5'-ATGATCCAGTGCTTGTTTCC-3') (SEQ ID No.4)

NIPBL-i16F (5'-TTGTCAGAGTTGCTGGAGATAC-3') (SEQ ID No.5)
NIPBL-i16R (5'-AATTTGATGGCACATAACTGTAG-3') (SEQ ID No.6)

ATP5A1-i3F (5'-AAATGGTTGAGTTCTCTTCTGG-3') (SEQ ID No.10)
ATP5A1-i3R (5'-TCAGGCTCCAAGTTCAAGG-3') (SEQ ID No.11)
Alternatively, *NIPBL* intron 16 can be amplified using the following primer pair:
NIPBL-i16F2 (5'-GTCAGCAGTTTGTCAGAGTTG-3') (SEQ ID No.12)
NIPBL-i16R2 (5'-CAGTCACTAATTTGATGGCAC-3') (SEQ ID No.13).

Experimental procedures
Genomic DNA was isolated from blood or muscle tissue using conventional phenol-chloroform extraction, whereas contour feathers were processed either via the QlAamp DNA Micro kit (Qiagen, Hilden) modified according to Bush (2005): DNA extraction technique for plucked body contour feathers, molted body contour feathers, and plucked chick down using the QIAamp® DNA Micro Kit. http://www.aviangenetics.com/DNA_Extraction_Protocols/plucked_contour_feathers.html or via rapid simple alkaline extraction following Malagó et al. (2002), BMC Biotechnol. 2:19. Each 25-µl PCR reaction contained 0.5 U ThermoPrime Taq DNA Polymerase (ABgene, Epsom), 75 mM Tris-HCl pH 8.8, 20 mM (NH₄)₂SO₄, 0.01 % (v/v) Tween® 20, 2.5 mM MgCl₂, 0.1 mM of each dNTP, 10 pmol of each primer and >5 ng of genomic DNA, and was carried out for 2 min at 94°C followed by 35 cycles of 30 s at 94°C, 30 s at 52°C and 80 s at 72°C. Subsequent to agarose gel electrophoresis, all PCR products were immediately purified or excised from agarose gels and then purified. Sequencing of the samples was conducted either directly using the specific PCR primers or indirectly via standard M13-F and M13-R primers after ligation into the pDrive Cloning Vector (Qiagen, Hilden) and electroporation into TOP10 cells (Invitrogen, Groningen).

Results
PCR amplifications of the *CHD1* intron 16 in neognaths (Fridolfsson & Ellegren, cited above), the *CHD1* intron 9 in neognaths and the *NIPBL* intron 16 in neoavians yielded two distinct amplicons in females and only the respective larger amplicon in males (Figure 7).
Consequently, to the same degree as shared retroposon insertions strongly indicate common ancestry in a gametologous gene tree, retroposon insertions in one of the two gametologs are ideal tools for molecular sexing (Hedges et al. (2003), Anal. Biochem. 312:77-79). Due to the distinct size differences in Z and W amplicons (200 bp in *CHD1* intron 16, 400 bp in *NIPBL* intron 16 and 500 bp in *CHD1* intron 9), the markers of the present invention are not prone to misinterpretation compared to previous markers based on short random indels (e.g., 50 bp in the marker reported by Griffiths et al., cited above).
Moreover, in Figure 3 the results of the comparison of sexing efficiency of *CHD1* intron 16 using previously published primers, i.e., "prior art" (Fridolfsson & Ellegren, cited above) or primers of the present invention (SEQ ID Nos. 3 and 4) are shown. Primers of the present invention reliably identify the sex of problematic species while prior art primers fail and thus lead to misidentifications.
In sum, these three universal and independent tests *(CHD1* introns 9 and 16, *NIPBL* intron 16) should enable ornithologists to conveniently determine molecular gender of virtually 95% to 99% of all extant birds.

## Claims

1. Pair of oligonucleotides selected from the group consisting of:
(a) a first oligonucleotide comprising at least 10 consecutive nucleotides of the sequence shown in SEQ ID No.1 and a second oligonucleotide comprising at least 10 consecutive nucleotides of the sequence shown in SEQ ID No.2;
(b) a first oligonucleotide comprising at least 10 consecutive nucleotides of the sequence shown in SEQ ID No.3 and a second oligonucleotide comprising at least 10 consecutive nucleotides of the sequence shown in SEQ ID No.4;
(c) a first oligonucleotide comprising at least 10 consecutive nucleotides of the sequence shown in SEQ ID No.5 and a second oligonucleotide comprising at least 10 consecutive nucleotides of the sequence shown in SEQ ID No.6; and
(d) a first oligonucleotide comprising at least 10 consecutive nucleotides of the sequence shown in SEQ ID No.12 and a second oligonucleotide comprising at least 10 consecutive nucleotides of the sequence shown in SEQ ID No.13,
wherein said pair of oligonucleotides is capable of amplifying a Z- and W-gametologous sequence from one or more species selected from the group consisting of *Chloebia gouldiae, Locustella ochotensis, Parus major, Parus palustris* and *Taeniopygia guttata.*

2. The pair of oligonucleotides of claim 1, wherein each of said oligonucleotides has a length of not more than 40 nucleotides.

3. The pair of oligonucleotides of any one of the preceding claims, wherein
(a) said first oligonucleotide as referred to in (a) comprises the nucleotide sequence shown in SEQ ID No.1 and said second oligonucleotide as referred to in (a) comprises the nucleotide sequence shown in SEQ ID No.2;
(b) said first oligonucleotide as referred to in (b) comprises the nucleotide sequence shown in SEQ ID No.3 and said second oligonucleotide as referred to in (b) comprises the nucleotide sequence shown in SEQ ID No.4;
(c) said first oligonucleotide as referred to in (c) comprises the nucleotide sequence shown in SEQ ID No.5 and said second oligonucleotide as referred to in (c) comprises the nucleotide sequence shown in SEQ ID No.6;
(d) said first oligonucleotide as referred to in (d) comprises the nucleotide sequence shown in SEQ ID No.12 and said second oligonucleotide as referred to in (d) comprises the nucleotide sequence shown in SEQ ID No.13

4. The pair of oligonucleotides of any one of the preceding claims, wherein said Z-gametologous sequence amplified by the pair of oligonucleotides
- of (a) comprises at least 10 consecutive nucleotides of the sequence shown in SEQ ID No.7 or 8;
- of (b) or (c) comprises at least 10 consecutive nucleotides of the sequence shown in SEQ ID No.9

5. The pair of oligonucleotides of any one of the preceding claims, wherein said oligonucleotides are further capable of amplifying a Z- and W-gametologous sequence from one or more avian species belonging to the group consisting of the following avian orders: Anseriformes, Apodiformes, Caprimulgiformes, Charadriiformes, Ciconiiformes, Coliiformes, Columbiformes, Coraciiformes, Cuculiformes, Falconiformes, Galbuliformes, Galliformes, Gaviiformes, Gruiformes, Opisthocomiformes, Passeriformes, Pelecaniformes, Piciformes, Phoenicopteriformes, Podicipediformes, Procellariiformes, Psittaciformes, Pterocliformes, Sphenisciformes, Strigiformes, Trogoniformes.

6. A method for the determination of the sex of an avian subject, comprising
(a) contacting a nucleotide sequence comprised in a sample from said subject with a pair of oligonucleotides referred to in any one of the preceding claims;
(b) amplifying said nucleotide sequence;
(c) determining the sex of said avian subject by the presence of amplification products of the same size or amplification products of two different sizes,
wherein amplification products of the same size are indicative of a male sex and amplification products of two different sizes are indicative of a female sex.

7. The method of claim 6, wherein said nucleotide sequence is amplified by an amplification technique selected from the group consisting of PCR, nested PCR, multiplex PCR, ligase chain reaction (LCR) , transcription-based amplification systems (TAS), self-sustained sequence replication (SSR), nucleic acid sequence-based amplification (NASBA), strand displacement amplification (SDA) or branched DNA (bDNA).

8. The method of claim 6 or 7, wherein in the amplification step a probe specific for one or more of the sequences shown in SEQ ID Nos.7-9 is present.

9. Use of a pair of oligonucleotides referred to in any one of the preceding claims for the determination of the sex of an avian subject.

10. A method for the identification of a pair of oligonucleotides for sexing of an avian subject, comprising the following steps:
(a) identifying Z-gametologous sequences which comprise CR1 retroposon and/or *hitchcock*-related LTR retroposon sequences;
(b) aligning said Z-gametologous sequences identified in (a) with corresponding W-gametologous sequences;
(c) determining whether said aligned W-gametologous sequences lack the CR1 and/or hitchcock-related LTR retroposon sequences; and
(d) providing a pair of oligonucleotides, wherein each oligonucleotide is substantially complementary to a region that is 5' and 3', respectively, of said aligned sequences and which is capable of simultaneously amplifying both a W-gametologous sequence and a Z-gametologous sequence, the latter comprising CR1 retroposon and/or *hitchcock*-related LTR retroposon sequence.

11. The method of claim 10, further comprising step (e) determining whether said pair of oligonucleotides is capable of amplifying a W-gametologous sequence from one or more species selected from the group consisting of *Chloebia gouldiae, Locustella ochotensis, Parus major, Parus palustris* and *Taeniopygia guttata.*

12. Pair of oligonucleotides for sexing of an avian subject obtainable by the method of claim 10 or 11.

13. A method for the identification of a probe for sexing of an avian subject, comprising:
(a) identifying Z-gametologous sequences which comprise CR1 retroposon and/or *hitchcock*-related LTR retroposon sequences;
(b) aligning said Z-gametologous sequences identified in (a) with corresponding W-gametologous sequences;
(c) determining whether said aligned W-gametologous sequences lack the CR1 and/or *hitchcock*-related LTR retroposon sequences; and
(d) providing a probe, wherein said probe is substantially complementary to a nucleotide sequence that spans the nucleotide sequence in the W gametolog that lacks the retroposon insertion in comparison to the Z gametolog.

14. A probe obtainable by the method of claim 13.

15. A kit for the determination of the sex of an avian subject comprising at least the pair of oligonucleotides of any one of claims 1-5 or 12 and/or a probe of claim 14.
